(19) Europäisches Patentamt
European Patent Office
Office européen des brevets

(11) EP 3 160 741 B1

(12) **EUROPEAN PATENT SPECIFICATION**

(45) Date of publication and mention of the grant of the patent:
**01.01.2025 Bulletin 2025/01**

(21) Application number: **15742148.8**

(22) Date of filing: **26.06.2015**

(51) International Patent Classification (IPC):
***B32B 27/06*** *(2006.01)* ***B32B 27/32*** *(2006.01)*
***C08F 210/16*** *(2006.01)* ***C08F 4/64*** *(2006.01)*
***C08L 23/08*** *(2006.01)* ***B32B 27/20*** *(2006.01)*

(52) Cooperative Patent Classification (CPC):
(C-Sets available)
**C08L 23/0815; B32B 5/00; B32B 5/02; B32B 5/022; B32B 7/00; B32B 27/06; B32B 27/18; B32B 27/20; B32B 27/28; B32B 27/30; B32B 27/306; B32B 27/308; B32B 27/32; B32B 27/325; C08J 5/18;** (Cont.)

(86) International application number:
**PCT/US2015/037870**

(87) International publication number:
**WO 2015/200741 (30.12.2015 Gazette 2015/52)**

# (54) BREATHABLE FILMS AND ARTICLES INCORPORATING SAME

ATMUNGSAKTIVEN FILME UND PRODUKTE ENTHALTEND DIESER FILME

FILMS PERMÉABLES À L'AIR ET ARTICLES COMPRENANT CES FILMS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2014 US 201462017525 P**

(43) Date of publication of application:
**03.05.2017 Bulletin 2017/18**

(60) Divisional application:
**24204937.7**

(73) Proprietor: **Dow Global Technologies LLC
Midland, MI 48674 (US)**

(72) Inventors:
- **WANG, Jian
Freeport, TX 77541 (US)**
- **JAIN, Pradeep
Freeport, TX 77541 (US)**
- **DEMIROURS, Mehmet
Freeport, TX 77541 (US)**
- **PATEL, Rajen M.
Freeport, TX 77541 (US)**
- **DEAVENPORT, Joseph L.
Freeport, TX 77541 (US)**
- **DEGROOT, Jacquelyn A.
Freeport, TX 77541 (US)**
- **GUERRA, Suzanne M.
Freeport, TX 77541 (US)**
- **SHAH, Viraj
Freeport, TX 77541 (US)**
- **BENSASON, Selim
CH-8810 Horgen (CH)**
- **OJHA, Satyajeet
Freeport, TX 77541 (US)**

(74) Representative: **Boult Wade Tennant LLP
Salisbury Square House
8 Salisbury Square
London EC4Y 8AP (GB)**

(56) References cited:
**EP-A1- 1 375 584
EP-A2- 2 374 822
WO-A1-2012/061168
WO-A1-2013/052636
WO-A1-2014/058639
US-A1- 2013 046 061**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

(52) Cooperative Patent Classification (CPC): (Cont.)
**C08L 23/0807;** B32B 2255/00; B32B 2255/02; B32B 2262/00; B32B 2262/02; B32B 2262/0253; B32B 2307/50; B32B 2307/514; B32B 2307/544; B32B 2307/718; B32B 2307/724; B32B 2307/726; C08J 2323/06; C08L 2314/02; C08L 2314/06

C-Sets
**C08F 210/16, C08F 2/06;**
**C08F 210/16, C08F 4/64193, C08F 4/6555;**
**C08L 23/0807, C08L 2314/02, C08L 2314/06;**
**C08L 23/0815, C08L 2314/02, C08L 2314/06;**
C08F 210/16, C08F 2500/10, C08F 2500/12, C08F 2500/19

## Description

### FIELD

The present invention relates generally to breathable films and to articles incorporating breathable films.

### INTRODUCTION

Breathable films are widely used in hygiene applications such as diaper backsheets. Persons of skill in the art generally understand breathable films to have a microporous morphology with some ability to allow the passage of moisture vapor. *See, e.g.,* Wu et al., "Novel Microporous Films and Their Composites," Journal of Engineered Fibers and Fabrics, Vol. 2, Issue 1, at 49-59 (2007).

Breathable films are typically made by incorporating 40 to 60% of a mineral filler, such as calcium carbonate ($CaCO_3$), into polyolefin resin, such as polyethylene or polypropylene or combinations of these materials, making a cast or blown film, and stretching or orienting the cast or blown film via machine direction orientation ("MDO") rolls, via tentering, or via intermeshing gears whereby the film is ringrolled, or incrementally stretched in one or both of the machine direction or cross direction below the melting point of the polyolefin resin. The breathability or water vapor transmission rate (WVTR) of the film is important for some applications, such as diaper backsheet films, protective clothing, surgical suits, and housewrap. In these applications, films can act as a liquid barrier while permitting the transmission of water vapor to provide benefits such as protection and comfort to the end-user in the case of hygiene and medical applications and protection from the elements without the accumulation of moisture in the case of housewrap.

For diaper backsheet applications, breathable films need a good balance of processability, stiffness and toughness. Processability, in terms of draw down capability, is needed during the extrusion and semi-solid state stretching steps of the process to ensure good, uniform draw down without breaks. Stiffness, often measured as film modulus, enables good dimension stability for films as they go through the high speed film printing and diaper making processes. This ultimately provides staple print repeat lengths and predictable web widths. Toughness is needed to prevent film puncture due to the presence of super absorbent polymer (SAP) particles in the absorbent core next to the film and to prevent leakers formed by hydrohead pressure due to a saturated absorbent core and the weight of the end-user.

While ethylene-based polymers have been used for breathable films, there remains a need for ethylene-based compositions that can be used in the manufacture of breathable films having desirable properties and related articles.

### SUMMARY

The present invention utilizes ethylene-based polymers exhibiting certain features in the formation of breathable films with desirable properties. For example, in some embodiments, the breathable films provide desirable processability, stiffness, toughness, and/or WVTR values for hygiene and other applications.

In one aspect, the present invention provides a breathable film comprising a layer formed from a composition comprising a first composition, wherein the first composition comprises at least one ethylene-based polymer and wherein the first composition comprises a MWCDI value greater than 0.9, and a melt index ratio (I10/I2) that meets the following equation: $I10/I2 \geq 7.0 - 1.2 \times \log(12)$.

These and other embodiments are described in more detail in the Detailed Description.

### BRIEF DESCRIPTION OF THE DRAWINGS

Figure 1 depicts the plot of "$SCB_f$ versus IR5 Area Ratio" for ten SCB Standards.
Figure 2 depicts the several GPC profiles for the determination of IR5 Height Ratio for Inventive First Composition 2.
Figure 3 depicts the plot of "$SCB_f$ versus Polyethylene Equivalent molecular Log $Mw_i$ (GPC)" for Inventive First Composition 2.
Figure 4 depicts a plot of the "Mole Percent Comonomer versus Polyethylene Equivalent Log $M_{wi}$ (GPC)" for Inventive First Composition 2.
Figure 5 depicts some GPC MWD profiles and corresponding comonomer distribution overlays for some inventive and comparative compositions (density 0.916-0.919 g/cc).
Figure 6 depicts some GPC MWD profiles and corresponding comonomer distribution overlays for some inventive and comparative compositions (density 0.924-0.926 g/cc).
Figure 7 depicts some GPC MWD profiles and corresponding comonomer distribution overlays for some inventive and comparative compositions (Cast stretch).

## DETAILED DESCRIPTION

It has been discovered that the inventive compositions can be used to form inventive breathable films and related products. Such compositions contain an ethylene-based polymer that has a superior comonomer distribution, which is significantly higher in comonomer concentration, and a good distribution of comonomer, in the high molecular weight polymer molecules, and is significantly lower in comonomer concentration in the low molecular weight polymer molecules, as compared to conventional polymers of the art at the same overall density. It has also been discovered that the ethylene-based polymer has low LCB (Long Chain Branches), as indicated by low ZSVR, as compared to conventional polymers. As a result of this optimized distribution of the comonomer, as well as the inherent low LCB nature, the inventive compositions have more tie chains, and thus, improved film toughness. The inventive compositions can be useful in forming the inventive breathable films and related products of the present invention.

The invention provides a composition comprising a first composition, comprising at least one ethylene-based polymer, wherein the first composition comprises a MWCDI value greater than 0.9, and a melt index ratio (I10/I2) that meets the following equation: $I10/I2 \geq 7.0 - 1.2 \times \log(I2)$.

The inventive composition may comprise a combination of two or more embodiments described herein.

The first composition may comprise a combination of two or more embodiments as described herein.

The ethylene-based polymer may comprise a combination of two or more embodiments as described herein.

In one embodiment, the first composition has a MWCDI value less than, or equal to, 10.0, further less than, or equal to, 8.0, further less than, or equal to, 6.0.

In one embodiment, the first composition has a MWCDI value less than, or equal to, 5.0, further less than, or equal to, 4.0, further less than, or equal to, 3.0.

In one embodiment, the first composition has a MWCDI value greater than, or equal to, 1.0, further greater than, or equal to, 1.1, further greater than, or equal to, 1.2.

In one embodiment, the first composition has a MWCDI value greater than, or equal to, 1.3, further greater than, or equal to, 1.4, further greater than, or equal to, 1.5.

In one embodiment, the first composition has a melt index ratio I10/I2 greater than, or equal to, 7.0, further greater than, or equal to, 7.1, further greater than, or equal to, 7.2, further greater than, or equal to, 7.3.

In one embodiment, the first composition has a melt index ratio I10/I2 less than, or equal to, 9.2, further less than, or equal to, 9.0, further less than, or equal to, 8.8, further less than, or equal to, 8.5.

In one embodiment, the first composition has a ZSVR value from 1.2 to 3.0, further from 1.2 to 2.5, further 1.2 to 2.0.

In one embodiment, the first composition has a vinyl unsaturation level greater than 10 vinyls per 1,000,000 total carbons. For example, greater than 20 vinyls per 1,000,000 total carbons, or greater than 50 vinyls per 1,000,000 total carbons, or greater than 70 vinyls per 1,000,000 total carbons, or greater than 100 vinyls per 1,000,000 total carbons.

In one embodiment, the first composition has a density in the range of 0.910 to 0.940 $g/cm^3$, for example from 0.910 to 0.935 $g/cm^3$, or from 0.910 to 0.930 $g/cm^3$, or from 0.910 to 0.925 $g/cm^3$. For example, the density can be from a lower limit of 0.910, 0.912, or 0.914 $g/cm^3$, to an upper limit of 0.925, 0.927, 0.930, or 0.935 $g/cm^3$ (1 $cm^3$ = 1 cc).

In one embodiment, the first composition has a melt index ($I_2$ or I2; at 190°C / 2.16kg) from 0.1 to 50 g/10 minutes, for example from 0.1 to 30 g/10 minutes, or from 0.1 to 20 g/10 minutes, or from 0.1 to 10 g/10 minutes. For example, the melt index ($I_2$ or I2; at 190°C / 2.16 kg) can be from a lower limit of 0.1, 0.2, or 0.5 g/10 minutes, to an upper limit of 1.0, 2.0, 3.0, 4.0, 5.0, 10, 15, 20, 25, 30, 40, or 50 g/10 minutes.

In one embodiment, the first composition has a molecular weight distribution, expressed as the ratio of the weight average molecular weight to number average molecular weight ($M_w/M_n$; as determined by conv. GPC) in the range of from 2.2 to 5.0. For example, the molecular weight distribution ($M_w/M_n$) can be from a lower limit of 2.2, 2.3, 2.4, 2.5, 3.0, 3.2, or 3.4, to an upper limit of 3.9, 4.0, 4.1, 4.2, 4.5, 5.0.

In one embodiment, the first composition has a number average molecular weight ($M_n$; as determined by conv. GPC) in the range from 10,000 to 50,000 g/mole. For example, the number average molecular weight can be from a lower limit of 10,000, 20,000, or 25,000 g/mole, to an upper limit of 35,000, 40,000, 45,000, or 50,000 g/mole.

In one embodiment, the first composition has a weight average molecular weight ($M_w$; as determined by conv. GPC) in the range from 70,000 to 200,000 g/mole. For example, the number average molecular weight can be from a lower limit of 70,000, 75,000, or 78,000 g/mole, to an upper limit of 120,000, 140,000, 160,000, 180,000 or 200,000 g/mole.

In one embodiment, the first composition has a melt viscosity ratio, Eta*0.1 / Eta*100, in the range from 2.2 to 7.0. For example, the number average molecular weight can be from a lower limit of 2.2, 2.3, 2.4 or 2.5, to an upper limit of 6.0, 6.2, 6.5, or 7.0.

In one embodiment, the ethylene-based polymer is an ethylene/$\alpha$-olefin interpolymer, and further an ethylene/$\alpha$-olefin copolymer.

In one embodiment, the first ethylene-based polymer is an ethylene/$\alpha$-olefin interpolymer, and further an ethylene/$\alpha$-olefin copolymer.

In one embodiment, the a-olefin has less than, or equal to, 20 carbon atoms. For example, the a-olefin comonomers may preferably have 3 to 10 carbon atoms, and more preferably 3 to 8 carbon atoms. Exemplary a-olefin comonomers include, but are not limited to, propylene, 1-butene, 1-pentene, 1-hexene, 1-heptene, 1-octene, 1-nonene, 1-decene, and 4-methyl-1-pentene. The one or more a-olefin comonomers may, for example, be selected from the group consisting of propylene, 1-butene, 1-hexene, and 1-octene; or in the alternative, from the group consisting of 1-butene, 1-hexene and 1-octene, and further 1-hexene and 1-octene.

In one embodiment, the ethylene-based polymer, or first ethylene-based polymer, has a molecular weight distribution ($M_w/M_n$; as determined by conv. GPC) in the range from 1.5 to 4.0, for example, from 1.5 to 3.5, or from 2.0 to 3.0. For example, the molecular weight distribution ($M_w/M_n$) can be from a lower limit of 1.5, 1.7, 2.0, 2.1, or 2.2, to an upper limit of 2.5, 2.6, 2.8, 3.0, 3.5, or 4.0.

In one embodiment, the first composition further comprises a second ethylene-based polymer. In a further embodiment, the second ethylene-based polymer is an ethylene/$\alpha$-olefin interpolymer, and further an ethylene/$\alpha$-olefin copolymer, or a LDPE.

In one embodiment, the a-olefin has less than, or equal to, 20 carbon atoms. For example, the a-olefin comonomers may preferably have 3 to 10 carbon atoms, and more preferably 3 to 8 carbon atoms. Exemplary a-olefin comonomers include, but are not limited to, propylene, 1-butene, 1-pentene, 1-hexene, 1-heptene, 1-octene, 1-nonene, 1-decene, and 4-methyl-1-pentene. The one or more a-olefin comonomers may, for example, be selected from the group consisting of propylene, 1-butene, 1-hexene, and 1-octene; or in the alternative, from the group consisting of 1-butene, 1-hexene and 1-octene, and further 1-hexene and 1-octene.

In one embodiment, the second ethylene-based polymer is a heterogeneously branched ethylene/$\alpha$-olefin interpolymer, and further a heterogeneously branched ethylene/$\alpha$-olefin copolymer. Heterogeneously branched ethylene/$\alpha$-olefin interpolymers and copolymers are typically produced using Ziegler/Natta type catalyst system, and have more comonomer distributed in the lower molecular weight molecules of the polymer.

In one embodiment, the second ethylene-based polymer has a molecular weight distribution ($M_w/M_n$) in the range from 3.0 to 5.0, for example from 3.2 to 4.6. For example, the molecular weight distribution ($M_w/M_n$) can be from a lower limit of 3.2, 3.3, 3.5, 3.7, or 3.9, to an upper limit of 4.6, 4.7, 4.8, 4.9, or 5.0.

In one embodiment, the composition comprises from 50 to 80 wt%, or from 50 to 85 wt%, or from 50 to 90 wt%, or from 50 to 95 wt% of the first composition, based on the weight of the composition.

In one embodiment, the composition comprises greater than, or equal to, 80 wt%, or greater than, or equal to, 85 wt%, or greater than, or equal to, 90 wt%, or greater than, or equal to, 95 wt%, or greater than, or equal to 98 wt% of the first composition, based on the weight of the composition.

In one embodiment, the composition further comprises another polymer. In a further embodiment, the polymer is selected from the following: a LLDPE, a VLDPE (a very low density polyethylene), a MDPE, a LDPE, a HDPE, a HMWHDPE (a high molecular weight HDPE), a propylene-based polymer, a polyolefin plastomer, a polyolefin elastomer, an olefin block copolymer, an ethylene vinyl acetate, an ethylene acrylic acid, an ethylene methacrylic acid, an ethylene methyl acrylate, an ethylene ethyl acrylate, an ethylene butyl acrylate, a polyisobutylene, a maleic anhydride-grafted polyolefin, an ionomer of any of the foregoing, or a combination thereof.

In one embodiment, the composition further comprises a LDPE. In a further embodiment, the LDPE is present in an amount from 5 to 50 wt%, further from 10 to 40 wt%, further from 15 to 30 wt%, based on the weight of the composition. In a further embodiment, the LDPE has a density from 0.915 to 0.930 g/cc, and a melt index (I2) from 0.15 to 30 g/10 min, further from 0.25 to 20 g/10 min.

In one embodiment, the composition further comprises one or more additives.

The invention also provides an article comprising at least one component formed from an inventive composition as described herein. In a further embodiment, the article is a film.

In some embodiments, the present invention relates to a breathable film formed from any of the inventive compositions as described herein. In some embodiments, a first composition (formed from an inventive composition described herein) in the breathable film may have an MWCDI value less than, or equal to 10.0. In some embodiments, the first composition used in the breathable film has a density of 0.910 to 0.950 g/cm$^3$ and/or a melt index (I2) of 0.5 to 30 g/10 minutes. The first composition used in the breathable film, in some embodiments, has a density of 0.915 to 0.940 g/cm$^3$. In some embodiments where the breathable film is a cast film, the melt index can be from 0.8 to 15 g/10 minutes, or from 1.5 to 5 g/10 minutes. In some embodiments where the breathable film is a blown film, the melt index can be from 0.7 to 1.5 g/10 minutes.

In some embodiments, the breathable film further comprises 40 to 65 percent by weight of a mineral filler (e.g., $CaCO_3$).

A breathable film, in some embodiments, has a basis weight of 10 to 20 g/m$^2$, or of 12 to 20 g/m$^2$, or of 8 to 18 g/m$^2$.

In some embodiments, the breathable film is a monolayer film. The breathable film, in some embodiments, is a multilayer film. In some embodiments, the breathabe film comprises up to 7 layers or, in the case of microlayer films, could comprise greater than 15 or 25 layers.

In some embodiments, a first layer of the breathable film can comprise, in addition to an inventive composition, a second polymer, wherein the second polymer is selected from the following: a LLDPE, a VLDPE, a LDPE, a MDPE, a HDPE, a HMWHDPE, a propylene-based polymer, a polyolefin plastomer, a polyolefin elastomer, an olefin block copolymer, an ethylene vinyl acetate, an ethylene acrylic acid, an ethylene methacrylic acid, an ethylene methyl acrylate, an ethylene ethyl acrylate, an ethylene butyl acrylate, a polyisobutylene, a maleic anhydride-grafted polyolefin, an ionomer of any of the foregoing, or a combination thereof.

In some embodiments where the breathable film is a multilayer film, the film can further comprise a second layer, wherein the second layer comprises a polymer selected from the following: the inventive composition, a LLDPE, a VLDPE (a very low density polyethylene), a MDPE, a LDPE, a HDPE, a HMWHDPE (a high molecular weight HDPE), a propylene-based polymer, a polyolefin plastomer, a polyolefin elastomer, an olefin block copolymer, an ethylene vinyl acetate, an ethylene acrylic acid, an ethylene methacrylic acid, an ethylene methyl acrylate, an ethylene ethyl acrylate, an ethylene butyl acrylate, an isobutylene, a maleic anhydride-grafted polyolefin, an ionomer of any of the foregoing, or a combination thereof.

The breathable film, in some embodiments, comprises at least 30 weight percent of any of the inventive compositions disclosed herein. In some embodiments, the breathable film comprises up to 60 weight percent of any of the inventive compositions disclosed herein. The breathable film, in some embodiments, comprises from 40 to 60 weight percent of an inventive composition, or from 45 to 55 weight percent of an inventive composition.

In some embodiments, a breathable film exhibits a water vapor transmission rate of at least 100 $g/m^2$-day-atm and up to 10,000 $g/m^2$-day-atm, preferably from 500 $g/m^2$-day-atm to 10,000 $g/m^2$-day-atm, or from 1,000 to 6,000 $g/m^2$-day-atm, or from 1,500 to 6,000 $g/m^2$-day-atm.

The breathable film, in some embodiments, exhibits a hydrohead of at least 60 cm as measured by EN 20811.

In some embodiments, the breathable film is oriented in at least the machine direction.

Some embodiments of the present invention relate to laminates comprising a breathable film as disclosed herein. In some such embodiments, the laminate may further comprise a non-woven material. Some embodiments of the present invention relate to an article comprising a laminate as disclosed herein.

Some embodiments of the present invention relate to an article comprising a breathable film as disclosed herein. Non-limiting examples of such articles can include backsheets for baby diapers, training pants, adult incontinence products, breathable barrier surgical gowns, housewrap, and filtration products.

## Polymerization

Polymerization processes include, but are not limited to, solution polymerization processes, using one or more conventional reactors, e.g., loop reactors, isothermal reactors, adiabatic reactors, stirred tank reactors, autoclave reactors in parallel, series, and/or any combinations thereof. The ethylene based polymer compositions may, for example, be produced via solution phase polymerization processes, using one or more loop reactors, adiabatic reactors, and combinations thereof.

In general, the solution phase polymerization process occurs in one or more well mixed reactors, such as one or more loop reactors and/or one or more adiabatic reactors at a temperature in the range from 115 to 250°C; for example, from 135 to 200°C, and at pressures in the range of from 300 to 1000 psig, for example, from 450 to 750 psig.

In one embodiment, the ethylene based polymer composition (e.g., the first composition of claim 1) may be produced in two loop reactors in series configuration, the first reactor temperature is in the range from 115 to 200°C, for example, from 135 to 165°C, and the second reactor temperature is in the range from 150 to 210°C, for example, from 185 to 200°C. In another embodiment, the ethylene based polymer composition may be produced in a single reactor, and the reactor temperature is in the range from 115 to 200°C, for example from 130 to 190°C. The residence time in a solution phase polymerization process is typically in the range from 2 to 40 minutes, for example from 5 to 20 minutes. Ethylene, solvent, one or more catalyst systems, optionally one or more cocatalysts, and optionally one or more comonomers, are fed continuously to one or more reactors. Exemplary solvents include, but are not limited to, isoparaffins. For example, such solvents are commercially available under the name ISOPAR E from ExxonMobil Chemical. The resultant mixture of the ethylene based polymer composition and solvent is then removed from the reactor or reactors, and the ethylene based polymer composition is isolated. Solvent is typically recovered via a solvent recovery unit, i.e., heat exchangers and separator vessel, and the solvent is then recycled back into the polymerization system.

In one embodiment, the ethylene based polymer composition may be produced, via a solution polymerization process, in a dual reactor system, for example a dual loop reactor system, wherein ethylene, and optionally one or more $\alpha$-olefins, are polymerized in the presence of one or more catalyst systems, in one reactor, to produce a first ethylene-based polymer, and ethylene, and optionally one or more $\alpha$-olefins, are polymerized in the presence of one or more catalyst systems, in a second reactor, to produce a second ethylene-based polymer. Additionally, one or more cocatalysts may be present.

In another embodiment, the ethylene based polymer composition may be produced via a solution polymerization

process, in a single reactor system, for example, a single loop reactor system, wherein ethylene, and optionally one or more α-olefins, are polymerized in the presence of one or more catalyst systems. Additionally, one or more cocatalysts may be present.

As discussed above, the invention provides a process to form a composition comprising at least two ethylene-based polymers, said process comprising the following:

polymerizing ethylene, and optionally at least one comonomer, in solution, in the presence of a catalyst system comprising a metal-ligand complex of Structure I, to form a first ethylene-based polymer; and

polymerizing ethylene, and optionally at least one comonomer, in the presence of a catalyst system comprising a Ziegler/Natta catalyst, to form a second ethylene-based polymer; and wherein Structure I is as follows:

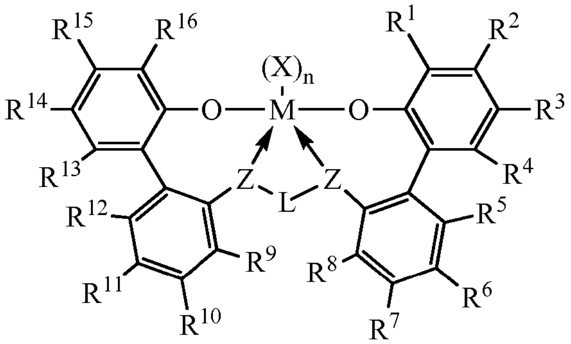

(I),

wherein:

M is titanium, zirconium, or hafnium, each, independently, being in a formal oxidation state of +2, +3, or +4; and

n is an integer from 0 to 3, and wherein when n is 0, X is absent; and

each X, independently, is a monodentate ligand that is neutral, monoanionic, or dianionic; or two Xs are taken together to form a bidentate ligand that is neutral, monoanionic, or dianionic; and

X and n are chosen, in such a way, that the metal-ligand complex of formula (I) is, overall, neutral; and

each Z, independently, is O, S, N($C_1$-$C_{40}$)hydrocarbyl, or P($C_1$-$C_{40}$)hydrocarbyl; and

wherein the Z-L-Z fragment is comprised of formula (1):

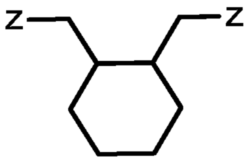

(1);

$R^1$ through $R^{16}$ are each, independently, selected from the group consisting of the following: a substituted or unsubstituted ($C_1$-$C_{40}$)hydrocarbyl, a substituted or unsubstituted ($C_1$-$C_{40}$)heterohydrocarbyl, $Si(R^C)_3$, $Ge(R^C)_3$, $P(R^P)_2$, $N(R^N)_2$, $OR^C$, $SR^C$, $NO_2$, CN, $CF_3$, $R^CS(O)$-, $R^CS(O)_2$-, $(R^C)_2C$=N-, $R^CC(O)O$-, $R^COC(O)$-, $R^CC(O)N(R)$-, $(R^C)_2NC(O)$-, halogen atom, hydrogen atom; and wherein each $R^C$ is independently a (C1-C30)hydrocarbyl; $R^P$ is a (C1-C30)hydrocarbyl; and $R^N$ is a (C1-C30)hydrocarbyl; and

wherein, optionally, two or more R groups (from $R^1$ through $R^{16}$) can combine together into one or more ring structures, with such ring structures each, independently, having from 3 to 50 atoms in the ring, excluding any hydrogen atom.

An inventive process may comprise a combination of two or more embodiments as described herein.

In one embodiment, said process comprises polymerizing ethylene, and optionally at least one α-olefin, in solution, in the presence of a catalyst system comprising a metal-ligand complex of Structure I, to form a first ethylene-based polymer; and polymerizing ethylene, and optionally at least one α-olefin, in the presence of a catalyst system comprising a Ziegler/Natta catalyst, to form a second ethylene-based polymer. In a further embodiment, each a-olefin is independently a C1-C8 α-olefin.

In one embodiment, optionally, two or more R groups from $R^9$ through $R^{13}$, or $R^4$ through $R^8$ can combine together into one or more ring structures, with such ring structures each, independently, having from 3 to 50 atoms in the ring, excluding any hydrogen atom.

In one embodiment, M is hafnium.

In one embodiment, Rand $R^{14}$ are each independently an alkyl, and further a C1-C3 alkyl, and further methyl.

In one embodiment, $R^1$ and $R^{16}$ are each as follows:

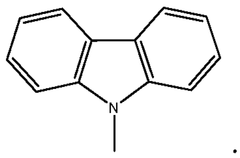

In one embodiment, each of the aryl, heteroaryl, hydrocarbyl, heterohydrocarbyl, $Si(R^C)_3$, $Ge(R^C)_3$, $P(R^P)_2$, $N(R^N)_2$, $OR^C$, $SR^C$, $R^CS(O)$-, $R^CS(O)_2$-, $(R^C)_2C$=N-, $R^CC(O)O$-, $R^COC(O)$-, $R^CC(O)N(R)$-, $(R^C)_2NC(O)$-, hydrocarbylene, and heterohydrocarbylene groups, independently, is unsubstituted or substituted with one or more $R^S$ substituents; and each $R^S$ independently is a halogen atom, polyfluoro substitution, perfluoro substitution, unsubstituted $(C_1$-$C_{18})$alkyl, $F_3C$-, $FCH_2O$-, $F_2HCO$-, $F_3CO$-, $R_3Si$-, $R_3Ge$-, RO-, RS-, RS(O)-, $RS(O)_2$-, $R_2P$-, $R_2N$-, $R_2C$=N-, NC-, RC(O)O-, ROC(O)-, RC(O)N(R)-, or $R_2NC(O)$-, or two of the $R^S$ are taken together to form an unsubstituted $(C_1$-$C_{18})$alkylene, wherein each R independently is an unsubstituted $(C_1$-$C_{18})$alkyl.

In one embodiment, two or more of $R^1$ through $R^{16}$ do not combine to form one or more ring structures.

In one embodiment, the catalyst system suitable for producing the first ethylene/$\alpha$-olefin interpolymer is a catalyst system comprising bis((2-oxoyl-3-(dibenzo-1H-pyrrole-1-yl)-5-(methyl)phenyl)-2-phenoxymethyl)-methylene-1,2-cyclohexanediylhafnium (IV) dimethyl, represented by the following Structure IA:

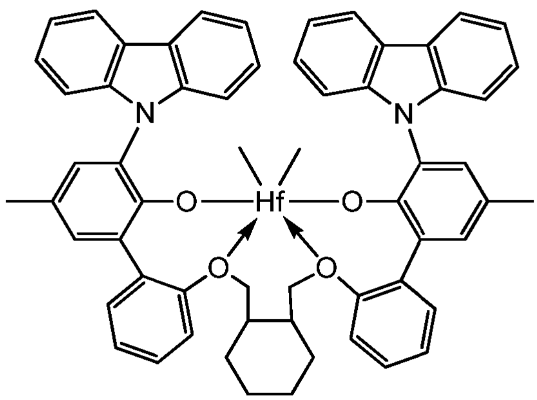

(IA).

The Ziegler/Natta catalysts suitable for use in the invention are typical supported, Ziegler-type catalysts, which are particularly useful at the high polymerization temperatures of the solution process. Examples of such compositions are those derived from organomagnesium compounds, alkyl halides or aluminum halides or hydrogen chloride, and a transition metal compound. Examples of such catalysts are described in U.S. Pat Nos. 4,612,300; 4,314,912; and 4,547,475; the teachings of which are incorporated herein by reference.

Particularly suitable organomagnesium compounds include, for example, hydrocarbon soluble dihydrocarbylmagnesium, such as the magnesium dialkyls and the magnesium diaryls. Exemplary suitable magnesium dialkyls include, particularly, n-butyl-sec-butylmagnesium, diisopropylmagnesium, di-n-hexylmagnesium, isopropyl-n-butyl-magnesium, ethyl-n-hexylmagnesium, ethyl-n-butylmagnesium, di-n-octylmagnesium, and others, wherein the alkyl has from 1 to 20 carbon atoms. Exemplary suitable magnesium diaryls include diphenylmagnesium, dibenzylmagnesium and ditolylmagnesium. Suitable organomagnesium compounds include alkyl and aryl magnesium alkoxides and aryloxides and aryl and alkyl magnesium halides, with the halogen-free organomagnesium compounds being more desirable.

Halide sources include active non-metallic halides, metallic halides, and hydrogen chloride. Suitable non-metallic halides are represented by the formula R'X, wherein R' is hydrogen or an active monovalent organic radical, and X is a halogen. Particularly suitable non-metallic halides include, for example, hydrogen halides and active organic halides, such as t-alkyl halides, allyl halides, benzyl halides and other active hydrocarbyl halides. By an active organic halide is meant a hydrocarbyl halide that contains a labile halogen at least as active, i.e., as easily lost to another compound, as the halogen of sec-butyl chloride, preferably as active as t-butyl chloride. In addition to the organic monohalides, it is understood that organic dihalides, trihalides and other polyhalides that are active, as defined hereinbefore, are also suitably employed. Examples of preferred active non-metallic halides, include hydrogen chloride, hydrogen bromide, t-butyl chloride, t-amyl bromide, allyl chloride, benzyl chloride, crotyl chloride, methylvinyl carbinyl chloride, a-phenylethyl bromide, diphenyl methyl chloride, and the like. Most preferred are hydrogen chloride, t-butyl chloride, allyl chloride and

benzyl chloride.

Suitable metallic halides include those represented by the formula MRy-a Xa, wherein: M is a metal of Groups IIB, IIIA or IVA of Mendeleev's periodic Table of Elements; R is a monovalent organic radical; X is a halogen; y has a value corresponding to the valence of M; and "a" has a value from 1 to y. Preferred metallic halides are aluminum halides of the formula $AlR_{3-a} X_a$, wherein each R is independently hydrocarbyl, such as alkyl; X is a halogen; and "a" is a number from 1 to 3. Most preferred are alkylaluminum halides, such as ethylaluminum sesquichloride, diethylaluminum chloride, ethylaluminum dichloride, and diethylaluminum bromide, with ethylaluminum dichloride being especially preferred. Alternatively, a metal halide, such as aluminum trichloride, or a combination of aluminum trichloride with an alkyl aluminum halide, or a trialkyl aluminum compound may be suitably employed.

Any of the conventional Ziegler-Natta transition metal compounds can be usefully employed, as the transition metal component in preparing the supported catalyst component. Typically, the transition metal component is a compound of a Group IVB, VB, or VIB metal. The transition metal component is generally, represented by the formulas: $TrX'_{4-q}$ (OR1) q, $TrX'_{4-q}$ (R2)q, $VOX'_3$ and $VO(OR)_3$.

Tr is a Group IVB, VB, or VIB metal, preferably a Group IVB or VB metal, preferably titanium, vanadium or zirconium; q is 0 or a number equal to, or less than, 4; X' is a halogen, and R1 is an alkyl group, aryl group or cycloalkyl group having from 1 to 20 carbon atoms; and R2 is an alkyl group, aryl group, aralkyl group, substituted aralkyls, and the like.

The aryl, aralkyls and substituted aralkys contain 1 to 20 carbon atoms, preferably 1 to 10 carbon atoms. When the transition metal compound contains a hydrocarbyl group, R2, being an alkyl, cycloalkyl, aryl, or aralkyl group, the hydrocarbyl group will preferably not contain an H atom in the position beta to the metal carbon bond. Illustrative, but non-limiting, examples of aralkyl groups are methyl, neopentyl, 2,2-dimethylbutyl, 2,2-dimethylhexyl; aryl groups such as benzyl; cycloalkyl groups such as 1-norbornyl. Mixtures of these transition metal compounds can be employed if desired.

Illustrative examples of the transition metal compounds include $TiCl_4$, $TiBr_4$, $Ti(OC_2H_5)_3Cl$, $Ti(OC_2H_5)Cl_3$, $Ti(OC_4H_9)_3Cl$, $Ti(OC_3H_7)_2Cl_2$, $Ti(OC_6H_{13})_2Cl_2$, $Ti(OC_8H_{17})_2Br_2$, and $Ti(OC_{12}H_{25})Cl_3$, $Ti(O\text{-}iC_3H_7)_4$, and $Ti(O\text{-}nC_4H_9)_4$. Illustrative examples of vanadium compounds include $VCl_4$, $VOCl_3$, $VO(OC_2H_5)_3$, and $VO(OC_4H_9)_3$. Illustrative examples of zirconium compounds include $ZrCl_4$, $ZrCl_3(OC_2H_5)$, $ZrCl_2(OC_2H_5)_2$, $ZrCl(OC_2H_5)_3$, $Zr(OC_2H_5)_4$, $ZrCl_3(OC_4H_9)$, $ZrCl_2(OC_4H_9)_2$, and $ZrCl(OC_4H_9)_3$.

An inorganic oxide support may be used in the preparation of the catalyst, and the support may be any particulate oxide, or mixed oxide which has been thermally or chemically dehydrated, such that it is substantially free of adsorbed moisture. See U. S. Pat Nos. 4,612,300; 4,314,912; and 4,547,475; the teachings of which are incorporated herein by reference.

In one embodiment, the composition comprises a MWCDI value greater than 0.9.

In one embodiment, the composition comprises a melt index ratio (I10/I2) that meets the following equation: $I10/I2 \geq 7.0 - 1.2 \times \log(I2)$.

The composition may comprise one embodiment, or a combination of two or more embodiments, as listed above for the "first composition."

An inventive process may comprise a combination of two or more embodiments described herein.

*Co-catalyst Component*

The above described catalyst systems can be rendered catalytically active by contacting it to, or combining it with, the activating co-catalyst, or by using an activating technique, such as those known in the art, for use with metal-based olefin polymerization reactions. Suitable activating co-catalysts, for use herein, include alkyl aluminums; polymeric or oligomeric alumoxanes (also known as aluminoxanes); neutral Lewis acids; and non-polymeric, non-coordinating, ion-forming compounds (including the use of such compounds under oxidizing conditions). A suitable activating technique is bulk electrolysis. Combinations of one or more of the foregoing activating co-catalysts and techniques are also contemplated. The term "alkyl aluminum" means a monoalkyl aluminum dihydride or monoalkylaluminum dihalide, a dialkyl aluminum hydride or dialkyl aluminum halide, or a trialkylaluminum. Aluminoxanes and their preparations are known at, for example, U. S. Patent 6,103,657. Examples of preferred polymeric or oligomeric alumoxanes are methylalumoxane, triisobutylaluminum-modified methylalumoxane, and isobutylalumoxane.

Exemplary Lewis acid activating co-catalysts are Group 13 metal compounds containing from 1 to 3 hydrocarbyl substituents as described herein. In some embodiments, exemplary Group 13 metal compounds are tri(hydrocarbyl)-substituted-aluminum or tri(hydrocarbyl)-boron compounds. In some other embodiments, exemplary Group 13 metal compounds are tri(hydrocarbyl)-substituted-aluminum or tri(hydrocarbyl)-boron compounds are tri(($C_1$-$C_{10}$)alkyl)aluminum or tri(($C_6$-$C_{18}$)aryl)boron compounds and halogenated (including perhalogenated) derivatives thereof. In some other embodiments, exemplary Group 13 metal compounds are tris(fluoro-substituted phenyl)boranes, in other embodiments, tris(pentafluorophenyl)borane. In some embodiments, the activating co-catalyst is a tris(($C_1$-$C_{20}$)hydrocarbyl) borate (e.g., trityl tetrafluoroborate) or a tri(($C_1$-$C_{20}$)hydrocarbyl)ammonium tetra(($C_1$-$C_{20}$)hydrocarbyl)borane (e.g., bis(octadecyl)methylammonium tetrakis(pentafluorophenyl)borane). As used herein, the term "ammonium" means a nitrogen

cation that is a $((C_1\text{-}C_{20})\text{hydrocarbyl})_4N^+$, a $((C_1\text{-}C_{20})\text{hydrocarbyl})_3N(H)^+$, a $((C_1\text{-}C_{20})\text{hydrocarbyl})_2N(H)_2^+$, $(C_1\text{-}C_{20})$ hydrocarbyl$N(H)_3^+$, or $N(H)_4^+$, wherein each $(C_1\text{-}C_{20})$hydrocarbyl may be the same or different.

Exemplary combinations of neutral Lewis acid activating co-catalysts include mixtures comprising a combination of a tri($(C_1\text{-}C_4)$alkyl)aluminum and a halogenated tri($(C_6\text{-}C_{18})$aryl)boron compound, especially a tris(pentafluorophenyl) borane. Other exemplary embodiments are combinations of such neutral Lewis acid mixtures with a polymeric or oligomeric alumoxane, and combinations of a single neutral Lewis acid, especially tris(pentafluorophenyl)borane with a polymeric or oligomeric alumoxane. Exemplary embodiments ratios of numbers of moles of (metal-ligand complex): (tris(pentafluoro-phenylborane): (alumoxane) [e.g., (Group 4 metal-ligand complex):(tris(pentafluoro-phenylborane):(alumoxane)] are from 1:1:1 to 1:10:30, other exemplary embodiments are from 1:1:1.5 to 1:5:10.

Many activating co-catalysts and activating techniques have been previously taught, with respect to different metal-ligand complexes, in the following U.S. patents: US 5,064,802; US 5,153,157; US 5,296,433; US 5,321,106; US 5,350,723; US 5,425,872; US 5,625,087; US 5,721,185; US 5,783,512; US 5,883,204; US 5,919,983; US 6,696,379; and US 7,163,907. Examples of suitable hydrocarbyloxides are disclosed in US 5,296,433. Examples of suitable Bronsted acid salts for addition polymerization catalysts are disclosed in US 5,064,802; US 5,919,983; US 5,783,512. Examples of suitable salts of a cationic oxidizing agent and a non-coordinating, compatible anion, as activating co-catalysts for addition polymerization catalysts, are disclosed in US 5,321,106. Examples of suitable carbenium salts as activating co-catalysts for addition polymerization catalysts are disclosed in US 5,350,723. Examples of suitable silylium salts, as activating co-catalysts for addition polymerization catalysts, are disclosed in US 5,625,087. Examples of suitable complexes of alcohols, mercaptans, silanols, and oximes with tris(pentafluorophenyl)borane are disclosed in US 5,296,433. Some of these catalysts are also described in a portion of US 6,515,155 B1, beginning at column 50, at line 39, and going through column 56, at line 55, only the portion of which is incorporated by reference herein.

In some embodiments, the above described catalyst systems can be activated to form an active catalyst composition by combination with one or more cocatalyst, such as a cation forming cocatalyst, a strong Lewis acid, or a combination thereof. Suitable cocatalysts for use include polymeric or oligomeric aluminoxanes, especially methyl aluminoxane, as well as inert, compatible, noncoordinating, ion forming compounds. Exemplary suitable cocatalysts include, but are not limited to, modified methyl aluminoxane (MMAO), bis(hydrogenated tallow alkyl)methyl, tetrakis(pentafluorophenyl)borate(1-) amine, triethyl aluminum (TEA), and any combinations thereof.

In some embodiments, one or more of the foregoing activating co-catalysts are used in combination with each other. In one embodiment, a combination of a mixture of a tri($(C_1\text{-}C_4)$hydrocarbyl)aluminum, tri($(C_1\text{-}C_4)$hydrocarbyl) borane, or an ammonium borate with an oligomeric or polymeric alumoxane compound, can be used.

**Additives, Additional Polymers and Applications**

An inventive composition may comprise one or more additives. Additives include, but are not limited to, antistatic agents, color enhancers, dyes, lubricants, fillers (for example, $TiO_2$ or $CaCO_3$), opacifiers, nucleators, processing aids, pigments, primary anti-oxidants, secondary anti-oxidants, UV stabilizers, anti-blocks, slip agents, tackifiers, fire retardants, anti-microbial agents, odor reducer agents, anti-fungal agents, and combinations thereof. In some embodiments, an inventive composition may comprise from about 0.001 to about 10 percent by the combined weight of such additives, based on the weight of the composition including such additives.

In breathable film applications (e.g., diaper, training pants, and adult incontinence backsheet films, surgical gowns, protective clothing, housewrap, etc.), large amounts of $CaCO_3$,or other mineral fillers, may be incorporated as a filler to increase WVTR and other properties related to breathability. For example, in some embodiments, a breathable film can comprise at least 20% by weight $CaCO_3$, or at least 30% by weight $CaCO_3$, or at least 40% by weight $CaCO_3$ based on the total weight of the film. In some embodiments, a breathable film can comprise up to 70% by weight $CaCO_3$, or up to 65 % by weight $CaCO_3$, or up to 60% by weight $CaCO_3$ based on the total weight of the film. A breathable film, in some embodiments can comprise from 20% to 70% by weight $CaCO_3$, or from 30% to 70% by weight $CaCO_3$, or from 40% to 65% by weight $CaCO_3$, or from 40% to 60% by weight $CaCO_3$ based on the total weight of the film.

An inventive composition may further comprise one or more other polymers. For example one or more other ethylene-based polymers (such polymers differ in one or more properties from the ethylene-based polymer of the first composition and the second ethylene-based polymer; i.e., density, melt index, comonomer, Mn, Mw, and/or MWD), or one or more propylene-based polymers, or combinations thereof.

Inventive compositions can be blended with other polymers to provide a resin in some embodiments. Non-limiting examples of other polymers that can blended with one or more inventive compositions include a LLDPE, a VLDPE, a LDPE, a MDPE, a HDPE, a HMWHDPE, a propylene-based polymer, a polyolefin plastomer, a polyolefin elastomer, an olefin block copolymer, an ethylene vinyl acetate, an ethylene acrylic acid, an ethylene methacrylic acid, an ethylene methyl acrylate, an ethylene ethyl acrylate, an ethylene butyl acrylate, a polyisobutylene, a maleic anhydride-grafted polyolefin, an ionomer of any of the foregoing, or a combination thereof. Such compositions may be blended via any method, known to a person of ordinary skill in the art, including, but not limited to, dry blending, and melt blending via any

suitable equipment, for example, an extruder. The resin further comprises a mineral filler, such as $CaCO_3$, to enable the microporous morphology and breathability that results from stretching the film comprising the resin. As noted in "The Role of Calcium Carbonate in Microporous Film Applications" by Deeba Ansara, Allison Calhoun, and Paul Merriman, PMA124PL, November 2001: "[Typically] stearic acid coated CaCO3 is used to enable free-flowing $CaCO_3$, that is easier to handle, compound, and disperse in the polymer. This coating results in a hydrophobic particle. The orientation process creates microvoids where the polymer separates from the calcium carbonate particles." Thus, in some embodiments using $CaCO_3$ as a mineral filler, the $CaCO_3$ can be coated with stearic acid. In some embodiments, the resin may comprise 45-80% $CaCO_3$ by weight based on the weight of the compound. In some such embodiments, the resin can provide a good dispersion of the $CaCO_3$ and can then be let down with other resins to provide a breathable film having a desired $CaCO_3$ concentration (e.g., 45-60% by weight $CaCO_3$).

In some embodiments, the present invention relates to a breathable film formed from any of the inventive compositions as described herein. In some embodiments, the breathable film is a monolayer film. The breathable film, in some embodiments, is a multilayer film. Breathable films can be formed from inventive compositions using methods and equipment well-known to those of skill in the art. For example, breathable films can be produced via a blown or cast film extrusion process and then stretched / oriented in a semi-solid state below the melting point of the polymer or polymer blend.

The amount of the inventive composition to use in breathable films of the present invention can depend on a number of factors including, for example, whether the film is a monolayer or multilayer film, the other layers in the film if it is a multilayer film, the desired properties of the film, the end use application of the film, the desired properties of the film, the equipment available to manufacture the film, and others. A breathable film of the present invention, in some embodiments, comprises at least 20 percent by weight of the inventive composition, or at least 30 percent by weight of the inventive composition, or at least 40 percent by weight of the inventive composition.

In embodiments where the breathable film comprises a multilayer film, the number of layers in the film can depend on a number of factors including, for example, the desired properties of the film, the desired thickness of the film, the content of the other layers of the film, whether any of the layers in the film are to be foamed, the end use application of the film, the equipment available to manufacture the film, and others. A multilayer breathable film can comprise up to 2, 3, 4, 5, 6, 7, 8, 9, 10, or 11 layers in various embodiments.

The inventive composition, in some embodiments, can be used in more than one layer of the film. Other layers within a multilayer film of the present invention can comprise, in various embodiments, a polymer selected from the following: the inventive composition, a LLDPE, a VLDPE (a very low density polyethylene), a MDPE, a LDPE, a HDPE, a HMWHDPE (a high molecular weight HDPE), a propylene-based polymer, a polyolefin plastomer, a polyolefin elastomer, an olefin block copolymer, an ethylene vinyl acetate, an ethylene acrylic acid, an ethylene methacrylic acid, an ethylene methyl acrylate, an ethylene ethyl acrylate, an ethylene butyl acrylate, an isobutylene, a maleic anhydride-grafted polyolefin, an ionomer of any of the foregoing, or a combination thereof. In some embodiments, a multilayer film of the present invention can comprise one or more tie layers known to those of skill in the art.

Breathable films can be coextrusions comprising two or more layers in some embodiments. Common film structures include, for example, a/b/a structures, a/b/c structures and others. Individual layers within a film structure can be 1% to 99% of the multilayer film based on the total weight of the film. Exemplary configurations include 10%/80%/10%, 20%/60%/20%, 1%/98%/1%, and others. Breathable films that are coextruded can be advantageous in some applications as they can, in some embodiments, achieve better bonding (e.g. thermal, adhesive, or ultrasonic) between the breathable film and another substrate, impart stiffness, and/or provide different haptics. In such multi-layer films, one or more of the additional layers (in addition to the layer comprising an inventive composition) can comprise a polymer selected from the following: an inventive composition, a LLDPE, a VLDPE, a LDPE, a MDPE, a HDPE, a HMWHDPE, a propylene-based polymer, a polyolefin plastomer, a polyolefin elastomer, an olefin block copolymer, an ethylene vinyl acetate, an ethylene acrylic acid, an ethylene methacrylic acid, an ethylene methyl acrylate, an ethylene ethyl acrylate, an ethylene butyl acrylate, a polyisobutylene, a maleic anhydride-grafted polyolefin, an ionomer of any of the foregoing, or combinations thereof.

As noted above, after formation of the film (e.g., via blown or cast film extrusion processes), the film is then stretched or oriented. Often, the film is heated prior to the stretching step to a temperature above its glass transition temperature, but below its melt temperature, such that it is stretched in a semi-solid state. Stretch methods include orientation in the machine direction or the use of ring rolling or intermeshing gears. In machine direction orientation (MDO), the film can then be heated via contact with heated rollers prior to being stretched between sets of rollers as is known to those of skill in the art. *See, e.g.,* P.C. Wu et al., "Novel Microporous Films and Their Composites," Journal of Engineered Fibers and Fabrics, Vol. 2, Issue 1, 49-59 (2007). The film is typically stretched between driven rollers whereby pairs of consecutive rollers are driven at progressively higher speeds to induce stretching *(see, e.g.,* Wu et al., p. 53 at FIGURE 7). Films are typically stretched from 1.5 to 5x their original length depending upon the properties desired. Higher levels of stretch impart larger pore sizes and higher levels of breathability in the film. Higher stretch can also impart splittiness or low tear properties in the film. Processability or stretch window is measured in terms of the minimum amount of stretch needed

to create a uniformly stretched film without the haze variations, often referred to as tiger striping, to the maximum amount of stretch that a film can withstand without creating pinholes or breaks in the web. Films with a larger stretch window can enable a wider range of performance properties and can be more resistant to pinholing and web breaks.

In orientation by ring rolling or with intermeshing gears (IMG), the film may also be heated to a temperature below its melting point via contact with heated rolls. The film is stretched between intermeshing rings or gears. The degree of stretching is controlled by the depth of engagement of an individual ring gear or ring relative to the adjacent ring(s) or gear(s). Such techniques are generally known to those of skill in the art. For example, U.S. Patents 4,116,892 and 4,153,751, which are incorporated herein by reference, provide additional information about ring rolling processes.

In both MDO and IMG processes, the films can be annealed after the stretching process. In some embodiments, the films can be stretched in 1 direction, such as with MDO or a single IMG step, of either machine direction stretching or cross direction stretching. Films can be stretched in two directions in series such as MDO followed by an IMG step to stretch in the cross direction. Another alternative is stretching in the machine direction via IMG followed by cross direction stretching via IMG. Yet another means of multi-direction stretching is via a tentering process.

Breathable films can have basis weights ranging from 8 to 24 grams per square meter or in the alternative, from 10 to 20 g/m$^2$ or in the alternative, from 12-18 g/m$^2$. The basis weight of the breathable film can depend on a number of factors including the desired properties of the film, the end use application of the film, the equipment available to manufacture the film, the cost allowed by the application, and other factors.

In some embodiments, a breathable film of the present invention can exhibit a water vapor transmission rate of at least 100 g/m$^2$-day-atm when measured in accordance with ASTM D-6701. A breathable film of the present invention, in some embodiments, can exhibit a water vapor transmission rate of at least 100 g/m$^2$-day-atm and up to 10,000 g/m$^2$-day-atm when measured in accordance with ASTM D-6701. In some embodiments, a breathable film of the present invention can exhibit a water vapor transmission rate of at least 500 g/m$^2$-day-atm and up to 10,000 g/m$^2$-day-atm when measured in accordance with ASTM D-6701. A breathable film of the present invention, in some embodiments, can exhibit a water vapor transmission rate of at least 1,000 g/m$^2$-day-atm and up to 6,000 g/m$^2$-day-atm when measured in accordance with ASTM D-6701. A breathable film of the present invention, in some embodiments, can exhibit a water vapor transmission rate of at least 1,500 g/m$^2$-day-atm and up to 6,000 g/m$^2$-day-atm when measured in accordance with ASTM D-6701.

In some embodiments, a breathable film of the present invention can exhibit a hydrohead of at least 60 cm as measured by EN 20811.

It is also contemplated that a breathable film may comprise additional layers, either coextruded, or as a laminate. These layers may be selected to provide additional functionality, for example, layers to provide extra strength, adhesion to another substrate such as a non-woven, and/or aesthetic properties such as feel or appearance.

Some embodiments of the present invention relate to laminates comprising one or more breathable films of the present invention. For example, breathable films of the present invention can be used in film/non-woven laminates. Typical non-wovens for use in such laminates can be spunlaid, airlaid, carded webs, or composities thereof. Typical non-woven composites for use in laminates with a breathable film of the present invention include three beams of spunbond, (e.g., S/S/S), a spunbond / meltblown / spunbond composite (e.g., S/M/S), and others. Common methods for joining the film to the non-wovens include, for example, bonded hot melt adhesive lamination, ultra-sonic bonding, and thermal bonding through a calendar or nip roll.

The present invention also relates to articles comprising at least one component formed from an inventive composition. The component can be, for example, a breathable film or film laminate. Such components can be used in disposable hygiene and medical products as liquid impermeable but breathable layers. Examples of articles comprising such breathable films or film laminates include diapers, training pants, feminine hygiene products, adult incontinence products, medical drapes, medical gowns, surgical suits, and others. In articles such as diaper, training pants, feminine hygiene products, and adult incontinence products, a breathable film or film laminate is also often referred to as a backsheet. In medical products, a breathable film or film laminate are often referred to as the "barrier layer" as the breathable film or film laminate can prevent contamination from a health care worker to a patient and vice versa. Breathable films can be incorporated into such articles using techniques known to those of skill in the art based on the teachings herein.

## DEFINITIONS

Unless stated to the contrary, implicit from the context, or customary in the art, all parts and percents are based on weight, and all test methods are current as of the filing date of this disclosure.

The term "composition," as used herein, includes material(s) which comprise the composition, as well as reaction products and decomposition products formed from the materials of the composition.

The term "comprising," and derivatives thereof, is not intended to exclude the presence of any additional component, step or procedure, whether or not the same is disclosed herein. In order to avoid any doubt, all compositions claimed herein through use of the term "comprising" may include any additional additive, adjuvant, or compound, whether

polymeric or otherwise, unless stated to the contrary. In contrast, the term, "consisting essentially of" excludes from the scope of any succeeding recitation any other component, step or procedure, excepting those that are not essential to operability. The term "consisting of" excludes any component, step or procedure not specifically delineated or listed.

The term "polymer," as used herein, refers to a polymeric compound prepared by polymerizing monomers, whether of the same or a different type. The generic term polymer thus embraces the term homopolymer (employed to refer to polymers prepared from only one type of monomer, with the understanding that trace amounts of impurities can be incorporated into the polymer structure), and the term interpolymer as defined hereinafter. Trace amounts of impurities may be incorporated into and/or within the polymer.

The term "interpolymer," as used herein, refers to a polymer prepared by the polymerization of at least two different types of monomers. The generic term interpolymer thus includes copolymers (employed to refer to polymers prepared from two different types of monomers), and polymers prepared from more than two different types of monomers.

The term, "olefin-based polymer," as used herein, refers to a polymer that comprises, in polymerized form, a majority amount of olefin monomer, for example ethylene or propylene (based on the weight of the polymer), and optionally may comprise at least one polymerized comonomer.

The term, "ethylene-based polymer," as used herein, refers to a polymer that comprises a majority amount of polymerized ethylene monomer (based on the total weight of the polymer), and optionally may comprise at least one polymerized comonomer.

The term, "ethylene/α-olefin interpolymer," as used herein, refers to an interpolymer that comprises, in polymerized form, a majority amount of ethylene monomer (based on the weight of the interpolymer), and at least one α-olefin.

The term, "ethylene/α-olefin copolymer," as used herein, refers to a copolymer that comprises, in polymerized form, a majority amount of ethylene monomer (based on the weight of the copolymer), and an α-olefin, as the only two monomer types.

The term "propylene-based polymer," as used herein, refers to a polymer that comprises, in polymerized form, a majority amount of propylene monomer (based on the total weight of the polymer) and optionally may comprise at least one polymerized comonomer.

TEST METHODS

**Melt index**

Melt indices $I_2$ (or I2) and $I_{10}$ (or I10) were measured in accordance to ASTM D-1238 (method B) at 190°C and at 2.16 kg and 10 kg load, respectively. Their values are reported in g/10 min.

**Density**

Samples for density measurement were prepared according to ASTM D4703. Measurements were made, according to ASTM D792, Method B, within one hour of sample pressing.

**Dynamic Shear Rheology**

Each sample was compression-molded into "3 mm thick × 25 mm diameter" circular plaque, at 177°C, for five minutes, under 10 MPa pressure, in air. The sample was then taken out of the press and placed on a counter top to cool.

Constant temperature, frequency sweep measurements were performed on an ARES strain controlled rheometer (TA Instruments), equipped with 25 mm parallel plates, under a nitrogen purge. For each measurement, the rheometer was thermally equilibrated, for at least 30 minutes, prior to zeroing the gap. The sample disk was placed on the plate, and allowed to melt for five minutes at 190°C. The plates were then closed to 2 mm, the sample trimmed, and then the test was started. The method had an additional five minute delay built in, to allow for temperature equilibrium. The experiments were performed at 190°C, over a frequency range from 0.1 to 100 rad/s, at five points per decade interval. The strain amplitude was constant at 10%. The stress response was analyzed in terms of amplitude and phase, from which the storage modulus (G'), loss modulus (G"), complex modulus (G*), dynamic viscosity (η* or Eta*), and tan δ (or tan delta) were calculated.

**Conventional Gel Permeation Chromatography (conv. GPC)**

A GPC-IR high temperature chromatographic system from PolymerChar (Valencia, Spain), was equipped with a Precision Detectors (Amherst, MA), 2-angle laser light scattering detector Model 2040, an IR5 infra-red detector and a 4-capillary viscometer, both from PolymerChar. Data collection was performed using PolymerChar Instrument Control software and data collection interface. The system was equipped with an on-line, solvent degas device and pumping

system from Agilent Technologies (Santa Clara, CA).

Injection temperature was controlled at 150 degrees Celsius. The columns used, were three, 10-micron "Mixed-B" columns from Polymer Laboratories (Shropshire, UK). The solvent used was 1,2,4-trichlorobenzene. The samples were prepared at a concentration of "0.1 grams of polymer in 50 milliliters of solvent." The chromatographic solvent and the sample preparation solvent each contained "200 ppm of butylated hydroxytoluene (BHT)." Both solvent sources were nitrogen sparged. Ethylene-based polymer samples were stirred gently at 160 degrees Celsius for three hours. The injection volume was "200 microliters,' and the flow rate was "1 milliliters/minute." The GPC column set was calibrated by running 21 "narrow molecular weight distribution" polystyrene standards. The molecular weight (MW) of the standards ranges from 580 to 8,400,000 g/mole, and the standards were contained in six "cocktail" mixtures. Each standard mixture had at least a decade of separation between individual molecular weights. The standard mixtures were purchased from Polymer Laboratories. The polystyrene standards were prepared at "0.025 g in 50 mL of solvent" for molecular weights equal to, or greater than, 1,000,000 g/mole, and at "0.050 g in 50 mL of solvent" for molecular weights less than 1,000,000 g/mole.

The polystyrene standards were dissolved at 80°C, with gentle agitation, for 30 minutes. The narrow standards mixtures were run first, and in order of decreasing "highest molecular weight component," to minimize degradation. The polystyrene standard peak molecular weights were converted to polyethylene molecular weight using Equation 1 (as described in Williams and Ward, J. Polym. Sci., Polym. Letters, 6, 621 (1968)):

$$\text{Mpolyethylene} = \text{A x (Mpolystyrene)}^{B} \quad \text{(Eqn. 1)},$$

where M is the molecular weight, A is equal to 0.4316 and B is equal to 1.0.

Number-average molecular weight (Mn(conv gpc)), weight average molecular weight (Mw-conv gpc), and z-average molecular weight (Mz(conv gpc)) were calculated according to Equations 2-4 below.

$$\text{Mn(conv gpc)} = \frac{\sum_{i=RV_{integration\ start}}^{i=RV_{integration\ end}} \left(IR_{measurement\ channel_i}\right)}{\sum_{i=RV_{integration\ start}}^{i=RV_{integration\ end}} \left( IR_{measurement\ channel_i} / M_{PE_i} \right)} \quad \text{(Eqn. 2)}$$

$$\text{Mw(conv gpc)} = \frac{\sum_{i=RV_{integration\ start}}^{i=RV_{integration\ end}} \left(M_{PE_i}\ IR_{measurement\ channel_i}\right)}{\sum_{i=RV_{integration\ start}}^{i=RV_{integration\ end}} \left(IR_{measurement\ channel_i}\right)} \quad \text{(Eqn. 3)}$$

$$\text{Mz(conv gpc)} = \frac{\sum_{i=RV_{integration\ start}}^{i=RV_{integration\ end}} \left(M_{PE_i}^{\ 2}\ IR_{measurement\ channel_i}\right)}{\sum_{i=RV_{integration\ start}}^{i=RV_{integration\ end}} \left(M_{PE_i}\ IR_{measurement\ channel_i}\right)} \quad \text{(Eqn. 4)}$$

In Equations 2-4, the RV is column retention volume (linearly-spaced), collected at "1 point per second," the IR is the baseline-subtracted IR detector signal, in Volts, from the IR5 measurement channel of the GPC instrument, and $M_{PE}$ is the polyethylene-equivalent MW determined from Equation 1. Data calculation were performed using "GPC One software (version 2.013H)" from PolymerChar.

**Creep Zero Shear Viscosity Measurement Method**

Zero-shear viscosities were obtained via creep tests, which were conducted on an AR-G2 stress controlled rheometer (TA Instruments; New Castle, Del), using "25-mm-diameter" parallel plates, at 190°C. The rheometer oven was set to test temperature for at least 30 minutes, prior to zeroing the fixtures. At the testing temperature, a compression molded sample disk was inserted between the plates, and allowed to come to equilibrium for five minutes. The upper plate was then lowered down to 50 μm (instrument setting) above the desired testing gap (1.5 mm). Any superfluous material was trimmed off, and the upper plate was lowered to the desired gap. Measurements were done under nitrogen purging, at a flow rate of 5 L/min. The default creep time was set for two hours. Each sample was compression-molded into a "2 mm thick × 25 mm diameter" circular plaque, at 177°C, for five minutes, under 10 MPa pressure, in air. The sample was then taken out of the press and placed on a counter top to cool.

A constant low shear stress of 20 Pa was applied for all of the samples, to ensure that the steady state shear rate was low enough to be in the Newtonian region. The resulting steady state shear rates were in the range from $10^{-3}$ to $10^{-4}$ $s^{-1}$ for the samples in this study. Steady state was determined by taking a linear regression for all the data, in the last 10% time window of the plot of "log (J(t)) vs. log(t)," where J(t) was creep compliance and t was creep time. If the slope of the linear regression was greater than 0.97, steady state was considered to be reached, then the creep test was stopped. In all cases in this study, the slope meets the criterion within one hour. The steady state shear rate was determined from the slope of the linear regression of all of the data points, in the last 10% time window of the plot of "ε vs. t," where ε was strain. The zero-shear viscosity was determined from the ratio of the applied stress to the steady state shear rate.

In order to determine if the sample was degraded during the creep test, a small amplitude oscillatory shear test was conducted before, and after, the creep test, on the same specimen from 0.1 to 100 rad/s. The complex viscosity values of the two tests were compared. If the difference of the viscosity values, at 0.1 rad/s, was greater than 5%, the sample was considered to have degraded during the creep test, and the result was discarded.

**Zero-Shear Viscosity Ratio** (ZSVR) is defined as the ratio of the zero-shear viscosity (ZSV) of the branched polyethylene material to the ZSV of a linear polyethylene material (see ANTEC proceeding below) at the equivalent weight average molecular weight (Mw(conv gpc)), according to the following Equation 5:

$$ZSVR = \frac{\eta_{0B}}{\eta_{0L}} = \frac{\eta_{0B}}{2.29^{-15} M_{w(conv \cdot gpc)}^{3.65}} \quad \text{(Eqn. 5).}$$

The ZSV value was obtained from creep test, at 190°C, via the method described above. The Mw(conv gpc) value was determined by the conventional GPC method (Equation 3), as discussed above. The correlation between ZSV of linear polyethylene and its Mw(conv gpc) was established based on a series of linear polyethylene reference materials. A description for the ZSV-Mw relationship can be found in the ANTEC proceeding: Karjala et al., Detection of Low Levels of Long-chain Branching in Polyolefins, Annual Technical Conference - Society of Plastics Engineers (2008), 66th 887-891.

## $^1$H NMR Method

A stock solution (3.26 g) was added to "0.133 g of the polymer sample" in 10 mm NMR tube. The stock solution was a mixture of tetrachloroethane-$d_2$ (TCE) and perchloroethylene (50:50, w:w) with 0.001M $Cr^{3+}$. The solution in the tube was purged with $N_2$, for 5 minutes, to reduce the amount of oxygen. The capped sample tube was left at room temperature, overnight, to swell the polymer sample. The sample was dissolved at 110°C with periodic vortex mixing. The samples were free of the additives that may contribute to unsaturation, for example, slip agents such as erucamide. Each **$^1$H** NMR analysis was run with a 10 mm cryoprobe, at 120°C, on Bruker AVANCE 400 MHz spectrometer.

Two experiments were run to get the unsaturation: the control and the double presaturation experiments. For the control experiment, the data was processed with an exponential window function with LB=1 Hz, and the baseline was corrected from 7 to -2 ppm. The signal from residual $^1$H of TCE was set to 100, and the integral $I_{total}$ from -0.5 to 3 ppm was used as the signal from whole polymer in the control experiment. The "number of $CH_2$ group, $NCH_2$," in the polymer was calculated as follows in Equation 1A:

$$NCH_2 = I_{total}/2 \quad \text{(Eqn. 1A).}$$

For the double presaturation experiment, the data was processed with an exponential window function with LB=1 Hz, and the baseline was corrected from about 6.6 to 4.5 ppm. The signal from residual $^1$H of TCE was set to 100, and the corresponding integrals for unsaturations ($I_{vinylene}$, $I_{trisubstituted}$, $I_{vinyl}$ and $I_{vinylidene}$) were integrated. It is well known to use NMR spectroscopic methods for determining polyethylene unsaturation, for example, see Busico, V., et al., Macromolecules, 2005, 38, 6988. The number of unsaturation unit for vinylene, trisubstituted, vinyl and vinylidene were calculated as follows:

$$N_{vinylene} = I_{vinylene}/2 \quad \text{(Eqn. 2A),}$$

$$N_{trisubstituted} = I_{trisubstitute} \quad \text{(Eqn. 3A),}$$

$$N_{vinyl} = I_{vinyl}/2 \quad \text{(Eqn. 4A),}$$

$$N_{vinylidene} = I_{vinylidene}/2 \quad \text{(Eqn. 5A)}.$$

The unsaturation units per 1,000 carbons, all polymer carbons including backbone carbons and branch carbons, were calculated as follows:

$$N_{vinylene}/1{,}000C = (N_{vinylene}/NCH_2)*1{,}000 \quad \text{(Eqn. 6A)},$$

$$N_{trisubstituted}/1{,}000C = (N_{trisubstituted}/NCH_2)*1{,}000 \quad \text{(Eqn. 7A)},$$

$$N_{vinyl}/1{,}000C = (N_{vinyl}/NCH_2)*1{,}000 \quad \text{(Eqn. 8A)},$$

$$N_{vinylidene}/1{,}000C = (N_{vinylidene}/NCH_2)*1{,}000 \quad \text{(Eqn. 9A)},$$

The chemical shift reference was set at 6.0 ppm for the $^1$H signal from residual proton from TCE-d2. The control was run with ZG pulse, NS=4, DS=12, SWH=10,000 Hz, AQ=1.64s, D1=14s. The double presaturation experiment was run with a modified pulse sequence, with O1P = 1.354 ppm, O2P = 0.960 ppm, PL9 = 57db, PL21 = 70 db, NS = 100, DS = 4, SWH = 10,000 Hz, AQ = 1.64s, D1 = 1 s (where D1 is the presaturation time), D13 = 13s. Only the vinyl levels were reported in Table 2 below.

### $^{13}$C NMR Method

Samples are prepared by adding approximately 3g of a 50/50 mixture of tetra-chloroethane-d2/orthodichlorobenzene, containing 0.025 M $Cr(AcAc)_3$, to a "0.25 g polymer sample" in a 10 mm NMR tube. Oxygen is removed from the sample by purging the tube headspace with nitrogen. The samples are then dissolved, and homogenized, by heating the tube and its contents to 150°C, using a heating block and heat gun. Each dissolved sample is visually inspected to ensure homogeneity.

All data are collected using a Bruker 400 MHz spectrometer. The data is acquired using a 6 second pulse repetition delay, 90-degree flip angles, and inverse gated decoupling with a sample temperature of 120°C. All measurements are made on non-spinning samples in locked mode. Samples are allowed to thermally equilibrate for 7 minutes prior to data acquisition. The 13C NMR chemical shifts were internally referenced to the EEE triad at 30.0 ppm.

C13 NMR Comonomer Content: It is well known to use NMR spectroscopic methods for determining polymer composition. ASTM D 5017-96; J. C. Randall et al., in "NMR and Macromolecules" ACS Symposium series 247; J. C. Randall, Ed., Am. Chem. Soc., Washington, D.C., 1984, Ch. 9; and J. C. Randall in "Polymer Sequence Determination", Academic Press, New York (1977) provide general methods of polymer analysis by NMR spectroscopy.

### Molecular Weighted Comonomer Distribution Index (MWCDI)

A GPC-IR, high temperature chromatographic system from PolymerChar (Valencia, Spain) was equipped with a Precision Detectors' (Amherst, MA) 2-angle laser light scattering detector Model 2040, and an IR5 infra-red detector (GPC-IR) and a 4-capillary viscometer, both from PolymerChar. The "15-degree angle" of the light scattering detector was used for calculation purposes. Data collection was performed using PolymerChar Instrument Control software and data collection interface. The system was equipped with an on-line, solvent degas device and pumping system from Agilent Technologies (Santa Clara, CA).

Injection temperature was controlled at 150 degrees Celsius. The columns used, were four, 20-micron "Mixed-A" light scattering columns from Polymer Laboratories (Shropshire, UK). The solvent was 1,2,4-trichlorobenzene. The samples were prepared at a concentration of "0.1 grams of polymer in 50 milliliters of solvent." The chromatographic solvent and the sample preparation solvent each contained "200 ppm of butylated hydroxytoluene (BHT)." Both solvent sources were nitrogen sparged. Ethylene-based polymer samples were stirred gently, at 160 degrees Celsius, for three hours. The injection volume was "200 microliters," and the flow rate was "1 milliliters/minute."

Calibration of the GPC column set was performed with 21 "narrow molecular weight distribution" polystyrene standards, with molecular weights ranging from 580 to 8,400,000 g/mole. These standards were arranged in six "cocktail" mixtures, with at least a decade of separation between individual molecular weights. The standards were purchased from Polymer Laboratories (Shropshire UK). The polystyrene standards were prepared at "0.025 grams in 50 milliliters of solvent" for molecular weights equal to, or greater than, 1,000,000 g/mole, and at "0.050 grams in 50 milliliters of solvent"

for molecular weights less than 1,000,000 g/mole. The polystyrene standards were dissolved at 80 degrees Celsius, with gentle agitation, for 30 minutes. The narrow standards mixtures were run first, and in order of decreasing "highest molecular weight component," to minimize degradation. The polystyrene standard peak molecular weights were converted to polyethylene molecular weights using Equation 1B (as described in Williams and Ward, J. Polym. Sci., Polym. Let., 6, 621 (1968)):

$$\text{Mpolyethylene} = \text{A x (Mpolystyrene)}^{\text{B}} \quad \text{(Eqn. 1B)},$$

where M is the molecular weight, A has a value of approximately 0.40 and B is equal to 1.0. The A value was adjusted between 0.385 and 0.425 (depending upon specific column-set efficiency), such that NBS 1475A (NIST) linear polyethylene weight-average molecular weight corresponded to 52,000 g/mole, as calculated by Equation 3B, below:

$$\text{Mn(LALS gpc)} = \frac{\sum_{i=RV_{integration\ start}}^{i=RV_{integration\ end}} \left(IR_{measurement\ channel_i}\right)}{\sum_{i=RV_{integration\ start}}^{i=RV_{integration\ end}} \left( IR_{measurement\ channel_i} / M_{PE_i} \right)} \quad \text{(Eqn. 2B)}$$

$$\text{Mw(LALS gpc)} = \frac{\sum_{i=RV_{integration\ start}}^{i=RV_{integration\ end}} \left(M_{PE_i}\ IR_{measurement\ channel_i}\right)}{\sum_{i=RV_{integration\ start}}^{i=RV_{integration\ end}} \left(IR_{measurement\ channel_i}\right)} \quad \text{(Eqn. 3B)}$$

In Equations 2B and 3B, RV is column retention volume (linearly-spaced), collected at "1 point per second." The IR is the baseline-subtracted IR detector signal, in Volts, from the measurement channel of the GPC instrument, and the $M_{PE}$ is the polyethylene-equivalent MW determined from Equation 1B. Data calculation were performed using "GPC One software (version 2.013H)" from PolymerChar.

A calibration for the IR5 detector ratios was performed using at least ten ethylene-based polymer standards (polyethylene homopolymer and ethylene/octene copolymers; narrow molecular weight distribution and homogeneous comonomer distribution) of known short chain branching (SCB) frequency (measured by the $^{13}C$ NMR Method, as discussed above), ranging from homopolymer (0 SCB/1000 total C) to approximately 50 SCB/1000 total C, where total C = carbons in backbone + carbons in branches. Each standard had a weight-average molecular weight from 36,000 g/mole to 126,000 g/mole, as determined by the GPC-LALS processing method described above. Each standard had a molecular weight distribution (Mw/Mn) from 2.0 to 2.5, as determined by the GPC-LALS processing method described above. Polymer properties for the SCB standards are shown in Table A.

Table A: "SCB" Standards

| Wt % Comonomer | IR5 Area ratio | SCB / 1000 Total C | Mw | Mw/Mn |
|---|---|---|---|---|
| 23.1 | 0.2411 | 28.9 | 37,300 | 2.22 |
| 14.0 | 0.2152 | 17.5 | 36,000 | 2.19 |
| 0.0 | 0.1809 | 0.0 | 38,400 | 2.20 |
| 35.9 | 0.2708 | 44.9 | 42,200 | 2.18 |
| 5.4 | 0.1959 | 6.8 | 37,400 | 2.16 |
| 8.6 | 0.2043 | 10.8 | 36,800 | 2.20 |
| 39.2 | 0.2770 | 49.0 | 125,600 | 2.22 |
| 1.1 | 0.1810 | 1.4 | 107,000 | 2.09 |
| 14.3 | 0.2161 | 17.9 | 103,600 | 2.20 |
| 9.4 | 0.2031 | 11.8 | 103,200 | 2.26 |

The "IR5 Area Ratio (or "IR5 $_{Methyl}$ Channel Area / IR5 Measurement Channel Area")" of "the baseline-subtracted area response of the IR5 methyl channel sensor" to "the baseline-subtracted area response of IR5 measurement channel

sensor" (standard filters and filter wheel as supplied by PolymerChar: Part Number IR5_FWM01 included as part of the GPC-IR instrument) was calculated for each of the "SCB" standards. A linear fit of the SCB frequency versus the "IR5 Area Ratio" was constructed in the form of the following Equation 4B:

SCB/1000 total C = $A_0$ + [$A_1$ × (IR5 Methyl Channel Area / IR5 Measurement Channel Area)] (Eqn. 4B), where $A_0$ is the "SCB/1000 total C" intercept at an "IR5 Area Ratio" of zero, and $A_1$ is the slope of the "SCB/1000 total C" versus "IR5 Area Ratio" and represents the increase in the "SCB/1000 total C" as a function of "IR5 Area Ratio."

A series of "linear baseline-subtracted chromatographic heights" for the chromatogram generated by the "IR5 methyl channel sensor" was established as a function of column elution volume, to generate a baseline-corrected chromatogram (methyl channel). A series of "linear baseline-subtracted chromatographic heights" for the chromatogram generated by the "IR5 measurement channel" was established as a function of column elution volume, to generate a baseline-corrected chromatogram (measurement channel).

The "IR5 Height Ratio" of "the baseline-corrected chromatogram (methyl channel)" to "the baseline-corrected chromatogram (measurement channel)" was calculated at each column elution volume index (each equally-spaced index, representing 1 data point per second at 1 ml/min elution) across the sample integration bounds. The "IR5 Height Ratio" was multiplied by the coefficient $A_1$, and the coefficient $A_0$ was added to this result, to produce the predicted SCB frequency of the sample. The result was converted into mole percent comonomer, as follows in Equation 5B:

$$\text{Mole Percent Comonomer} = \{SCB_f / [SCB_f + ((1000 - SCB_f * \text{Length of comonomer}) / 2)]\} * 100 \quad \text{(Eqn. 5B)},$$

where "$SCB_f$" is the "SCB per 1000 total C" and the "Length of comonomer" = 8 for octene, 6 for hexene, and so forth.

Each elution volume index was converted to a molecular weight value ($Mw_i$) using the method of Williams and Ward (described above; Eqn. 1B). The "Mole Percent Comonomer (y *axis)"* was plotted as a function of Log($Mw_i$), and the slope was calculated between $Mw_i$ of 15,000 and $Mw_i$ of 150,000 g/mole (end group corrections on chain ends were omitted for this calculation). An EXCEL linear regression was used to calculate the slope between, and including, $Mw_i$ from 15,000 to 150,000 g/mole. This slope is defined as the molecular weighted comonomer distribution index (MWCDI = Molecular Weighted Comonomer Distribution Index).

*Representative Determination of MWCDI (Inventive First Composition 2)*

A plot of the measured "SCB per 1000 total C (= $SCB_f$)" versus the observed "IR5 Area Ratio" of the SCB standards was generated (see Figure 1), and the intercept ($A_0$) and slope ($A_1$) were determined. Here, $A_0$ = -90.246 SCB/1000 total C; and $A_1$ = 499.32 SCB/1000 total C.

The "IR5 Height Ratio" was determined for Inventive Example 2 (see integration shown in Figure 2). This height ratio (IR5 Height Ratio of Inventive Example 2) was multiplied by the coefficient $A_1$, and the coefficient $A_0$ was added to this result, to produce the predicted SCB frequency of this example, at each elution volume index, as described above ($A_0$ = -90.246 SCB/1000 total C; and $A_1$ = 499.32 SCB/1000 total C). The $SCB_f$ was plotted as a function of polyethylene-equivalent molecular weight, as determined using Equation 1B, as discussed above. See Figure 3 (Log Mwi used as the x-axis).

The $SCB_f$ was converted into "Mole Percent Comonomer" via Equation 5B. The "Mole Percent Comonomer" was plotted as a function of polyethylene-equivalent molecular weight, as determined using Equation 1B, as discussed above. See Figure 4 (Log Mwi used as the x-axis). A linear fit was from Mwi of 15,000 g/mole to Mwi of 150,000 g/mole, yielding a slope of "2.27 mole percent comonomer × mole/g." Thus, the MWCDI = 2.27. An EXCEL linear regression was used to calculate the slope between, and including, Mwi from 15,000 to 150,000 g/mole.

**Film Testing Conditions**

The following physical properties were measured on the films produced (see experimental section).

- **45° Gloss:** ASTM D-2457.
- **Clarity:** ASTM: D-1746.

**ASTM D1003 Total Haze**

Samples measured for internal haze and overall (total) haze were sampled and prepared according to ASTM D1003. Internal haze was obtained via refractive index matching using mineral oil on both sides of the films. A Hazeguard

Plus (BYK-Gardner USA; Columbia, MD) was used for testing. Surface haze was determined as the difference between total haze and internal haze. The total haze was reported as the average of five measurements.

**ASTM D1922 MD (Machine Direction) and CD (Cross Direction) Elmendorf Tear Type B**

The Elmendorf Tear test determines the average force to propagate tearing through a specified length of plastic film or non rigid sheeting, after the tear has been started, using an Elmendorf-type tearing tester.
After film production from the sample to be tested, the film was conditioned for at least 40 hours at 23°C (+/- 2°C) and 50% R.H (+/- 5), as per ASTM standards. Standard testing conditions were 23°C (+/- 2°C) and 50% R.H (+/- 5), as per ASTM standards.
The force, in grams, required to propagate tearing across a film or sheeting specimen was measured using a precisely calibrated pendulum device. In the test, acting by gravity, the pendulum swung through an arc, tearing the specimen from a precut slit. The specimen was held on one side by the pendulum and on the other side by a stationary member. The loss in energy by the pendulum was indicated by a pointer or by an electronic scale. The scale indication was a function of the force required to tear the specimen.
The sample specimen geometry used in the Elmendorf tear test was the 'constant radius geometry' as specified in ASTM D1922. Testing is typically carried out on specimens that have been cut from both the film MD and CD directions. Prior to testing, the film specimen thickness was measured at the sample center. A total of 15 specimens per film direction were tested, and the average tear strength and average thickness reported. The average tear strength was normalized to the average thickness.

**ASTM D882 MD and CD, 1% and 2% Secant Modulus, Tensile Strength, and Break Strain**

The film MD (Machine Direction) and CD (Cross Direction) secant modulus and tensile break strength (or tensile strength) were measured with an Instron universal tester according to ASTM D882-10. The reported secant modulus value was the average of five measurements. The tensile break strength was determined using five film samples in each direction, with each sample being "1 inch × 6 inches" in size. The break strength is the tensile load or force required to rupture or break a given material. It is measured as pounds of force for each square inch (psi). The break strain is the strain at which the material breaks and is measured as length at break divided by original length multiplied by 100 (%).

**Puncture Strength**

The Puncture test determines the resistance of a film to the penetration of a probe at a standard low rate, a single test velocity. The puncture test method is based on ASTM D5748. After film production, the film was conditioned for at least 40 hours at 23°C (+/- 2°C) and 50% R.H (+/-5), as per ASTM standards. Standard testing conditions were 23°C (+/- 2°C) and 50% R.H (+/- 5), as per ASTM standards. Puncture was measured on a tensile testing machine. Square specimens were cut from a sheet to a size of "6 inches by 6 inches." The specimen was clamped in a "4 inch diameter" circular specimen holder, and a puncture probe was pushed into the centre of the clamped film at a cross head speed of 10 inches/minute. The internal test method follows ASTM D5748, with one modification. It deviated from the ASTM D5748 method, in that the probe used was a "0.5 inch diameter" polished steel ball on a "0.25 inch" support rod (rather than the 0.75 inch diameter, pear shaped probe specified in D5748).
There was a "7.7 inch" maximum travel length to prevent damage to the test fixture. There was no gauge length; prior to testing, the probe is as close as possible to, but not touching the specimen.
A single thickness measurement was made in the centre of the specimen. For each specimen, the maximum force, the force at break, the penetration distance, and the energy to break were determined. A total of five specimens were tested to determine an average puncture value. The puncture probe was cleaned using a "Kim-wipe" after each specimen.

**ASTM D1709 Dart Drop**

The film Dart Drop test determines the energy that causes a plastic film to fail, under specified conditions of impact by a free falling dart. The test result is the energy, expressed in terms of the weight of the missile falling from a specified height, which would result in the failure of 50% of the specimens tested.
After the film was produced, it was conditioned for at least 40 hours at 23°C (+/- 2°C) and 50% R.H (+/- 5), as per ASTM standards. Standard testing conditions are 23°C (+/- 2°C) and 50% R.H (+/- 5), as per ASTM standards.
The test result was reported as either by Method A, which uses a 1.5" diameter dart head and 26" drop height, or by Method B, which uses a 2" diameter dart head and 60" drop height. The sample thickness was measured at the sample center, and the sample then clamped by an annular specimen holder with an inside diameter of 5 inches. The dart was loaded above the center of the sample, and released by either a pneumatic or electromagnetic mechanism.

Testing was carried out according to the 'staircase' method. If the sample failed, a new sample was tested with the weight of the dart reduced by a known and fixed amount. If the sample did not fail, a new sample was tested with the weight of the dart increased by a known amount. After 20 specimens had been tested, the number of failures was determined. If this number was 10, then the test is complete. If the number was less than 10, then the testing continued, until 10 failures had been recorded. If the number was greater than 10, testing continued, until the total of non-failures was 10. The Dart Drop strength was determined from these data, as per ASTM D1709, and expressed in grams, as either the Dart Drop Impact of Type A or Type B. In some cases, the sample Dart Drop Impact strength may lie between A and B. In these cases, it was not possible to obtain a quantitative dart value.

**Water Vapor Transmission Rate (WVTR)**

Water Vapor Transmission Rate (or WVTR) is the absolute transmission rate. WVTR was determined according to ASTM D-6701 and is reported in units of $g/m^2/day$.

EXAMPLES

The following examples illustrate the present invention, but are not intended to limit the scope of the invention.

EXAMPLE 1:

**Inventive First Compositions 1, 2 and 3**

Inventive first compositions 1, 2 and 3, each contain two ethylene-octene copolymers. Each composition was prepared, via solution polymerization, in a dual series loop reactor system according to U.S. Pat. No. 5,977,251 (see Figure 2 of this patent), in the presence of a first catalyst system, as described below, in the first reactor, and a second catalyst system, as described below, in the second reactor.

The first catalyst system comprised a bis((2-oxoyl-3-(dibenzo-1H-pyrrole-1-yl)-5-(methyl)phenyl)-2-phenoxymethyl)-methylene-1,2-cyclohexanediylhafnium (IV) dimethyl, represented by the following formula (CAT 1):

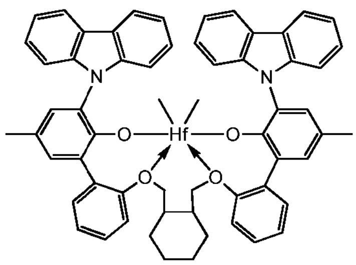

(CAT 1).

The molar ratios of the metal of CAT 1, added to the polymerization reactor, in-situ, to that of Cocat1 (modified methyl aluminoxane), or Cocat2 (bis(hydrogenated tallow alkyl)methyl, tetrakis(pentafluorophenyl)borate(1-) amine), are shown in Table 1.

The second catalyst system comprised a Ziegler-Natta type catalyst (CAT 2). The heterogeneous Ziegler-Natta type catalyst-premix was prepared substantially according to U.S. Pat. No. 4,612,300, by sequentially adding to a volume of ISOPAR E, a slurry of anhydrous magnesium chloride in ISOPAR E, a solution of $EtAlCl_2$ in heptane, and a solution of $Ti(O\text{-}iPr)_4$ in heptane, to yield a composition containing a magnesium concentration of 0.20M, and a ratio of Mg/Al/Ti of 40/12.5/3. An aliquot of this composition was further diluted with ISOPAR-E to yield a final concentration of 500 ppm Ti in the slurry. While being fed to, and prior to entry into, the polymerization reactor, the catalyst premix was contacted with a dilute solution of EtsAl, in themolar Al to Ti ratio specified in Table 1, to give the active catalyst.

The polymerization conditions for the inventive first compositions 1, 2 and 3 are reported in Table 1. As seen in Table 1, Cocat. 1 (modified methyl aluminoxane (MMAO)); and Cocat. 2 (bis(hydrogenated tallow alkyl)methyl, tetrakis(pentafluorophenyl)borate(1-) amine) were each used as a cocatalyst for CAT 1. Additional properties of the inventive compositions 1, 2 and 3 were measured, and are reported in Table 2. The GPC MWD profiles, and corresponding comonomer distribution overlays, are shown in Figures 5-7. Each polymer composition was stabilized with minor (ppm) amounts of stabilizers.

**Comparative First Compositions A and B**

Comparative compositions A and B, each contain two ethylene-octene copolymers, and each was prepared, via solution polymerization, in a dual loop reactor system, in the presence of a first catalyst system, as described below, in the first reactor, and a second catalyst system, as described below, in the second reactor. The first catalyst system comprised titanium, [*N*-(1,1-dimethylethyl)-1,1-dimethyl-1-[(1,2,3,3a,8a-η)-1,5,6,7-tetrahydro-2-methyl-*s*-indacen-1-yl]silanaminato(2-)-κ*N*][(1,2,3,4-η)-1,3-pentadiene]- (CAT 3, a constrained geometry catalyst). The second catalyst system comprised the Ziegler-Natta premix (CAT 2), as discussed above.

The polymerization conditions for comparative compositions A and B are reported in Table 1. As seen in Table 1, Cocat. 1 (modified methyl aluminoxane (MMAO)) and Cocat. 2 (bis(hydrogenated tallow alkyl)methyl, tetrakis(pentafluorophenyl)borate(1-) amine) were each used as cocatalyst for CAT 3. Additional properties of the comparative compositions A and B were measured, and are reported in Table 2. The GPC MWD profiles, and corresponding comonomer distribution overlays, are shown in Figures 5 and 6. Each polymer composition was stabilized with minor (ppm) amounts of stabilizers.

**Comparative C (First Composition)**

Comparative C is an ethylene-hexene copolymer composition, commercially available under the commercial designation EXCEED 1018CA from EXXONMOBIL Chemical Company, and having a density of approximately 0.918 g/cm$^3$, a melt index ($I_2$ or I2), measured at 190°C and 2.16 kg, of approximately 1.0 g/10 minutes. Additional properties of the comparative example C were measured, and are reported in Table 2. The GPC MWD profile, and corresponding comonomer distribution overlay, is shown in Figure 5.

**Comparative D (First Composition)**

Comparative D is an ethylene-octene copolymer composition, provided by The Dow Chemical Company, under the commercial designation ELITE 5230G, and having a density of approximately 0.916 g/cm$^3$, a melt index ($I_2$ or I2), measured at 190°C and 2.16 kg, of approximately 4.0 g/10 minutes. Additional properties of the comparative example D were measured, and are reported in Table 2. The GPC MWD profile, and corresponding comonomer distribution overlay, is shown in Figure 7.

Table 1: Polymerization Conditions (Rx1 = reactor 1; Rx2 = reactor 2)

| Sample # | Units | Inv. First 1 | Inv. First 2 | Inv. First 3 | Comp. First A | Comp. First B |
|---|---|---|---|---|---|---|
| Reactor Configuration | | Dual Series | Dual Series | Dual Series | Dual Series | Dual Series |
| Comonomer | | 1-octene | 1-octene | 1-octene | 1-octene | 1-octene |
| *REACTOR FEEDS* | | | | | | |
| First Reactor Total Solvent Flow | lb/hr | 1122 | 1057 | 1177 | 958 | 1061 |
| First Reactor Total Ethylene Flow | lb/hr | 190 | 175 | 269 | 184 | 187 |
| First Reactor Total Comonomer Flow | lb/hr | 74 | 48 | 118 | 97 | 58 |
| First Reactor Hydrogen Feed Flow | SCCM | 6827 | 5017 | 22848 | 525 | 857 |
| Second Reactor Total Solvent Flow | lb/hr | 384 | 451 | 421 | 494 | 561 |
| Second Reactor Total Ethylene Flow | lb/hr | 173 | 204 | 155 | 182 | 216 |
| Second Reactor Total Comonomer Flow | lb/hr | 12 | 8 | 22 | 50 | 17 |
| Second Reactor Hydrogen Feed Flow | SCCM | 298 | 99 | 100 | 2446 | 3829 |
| *REACTION* | | | | | | |

(continued)

| Sample # | Units | Inv. First 1 | Inv. First 2 | Inv. First 3 | Comp. First A | Comp. First B |
|---|---|---|---|---|---|---|
| Reactor Configuration | | Dual Series | Dual Series | Dual Series | Dual Series | Dual Series |
| Comonomer | | 1-octene | 1-octene | 1-octene | 1-octene | 1-octene |
| First Reactor Control Temperature | °C | 140 | 150 | 143 | 145 | 135 |
| First Reactor Ethylene Conversion | % | 86.7 | 90.5 | 72.7 | 69.4 | 77.7 |
| First Reactor Viscosity | cP | 2400 | 2315 | 824 | 891 | 1318 |
| Second Reactor Control Temperature | °C | 195 | 195 | 190 | 190 | 195 |
| Second Reactor Ethylene Conversion | % | 87.1 | 86 | 87.8 | 89.2 | 88.8 |
| Second Reactor Viscosity | cP | 869 | 876 | 264 | 892 | 848 |
| *CATALYST* | | | | | | |
| First Reactor Catalyst | type | CAT 1 | CAT 1 | CAT 1 | CAT 3 | CAT 3 |
| First Reactor Catalyst Efficiency | g polymer per g catalyst metal | 3,681,068 | 2,333,579 | 481,051 | 2,984,071 | 2,653,724 |
| First Reactor Cocatalyst (Cocat. 2) to Catalyst Metal Molar Ratio | Ratio | 1.3 | 1.8 | 1.2 | 1.2 | 1.5 |
| First Reactor Cocatalyst (Cocat. 1) to Catalyst Metal Molar Ratio | Ratio | 20 | 100 | 5 | 15 | 25 |
| Second Reactor Catalyst Efficiency | g polymer per g catalyst metal | 404,385 | 469,511 | 176,500 | 561,063 | 390,994 |
| Second Reactor Al to Ti Molar Ratio | Ratio | 4.0 | 4.0 | 1.2 | 4.0 | 4.0 |
| * solvent = ISOPAR E | | | | | | |

Table 2: Properties of Inventive and Comparative Compositions

| | Unit | Inv. First 1 | Inv. First 2 | Inv. First 3 | Comp. First A | Comp. First B | Comp. First C | Comp. First D |
|---|---|---|---|---|---|---|---|---|
| Density | g/cc | 0.9174 | 0.9245 | 0.9148 | 0.9162 | 0.9253 | 0.9191 | 0.9158 |
| $I_2$ | g/10 min | 0.83 | 0.87 | 3.91 | 0.93 | 0.80 | 0.95 | 4.05 |
| $I_{10}/I_2$ | | 7.7 | 8.0 | 7.3 | 8.2 | 8.4 | 6.0 | 7.0 |
| 7.0 - 1.2xlog(I2) | | 7.1 | 7.1 | 6.3 | 7.0 | 7.1 | 7.0 | 6.3 |
| Mn (conv.gpc) | g/mol | 32,973 | 33,580 | 20,244 | 33,950 | 34,626 | 45,645 | 26,355 |
| Mw (conv.gpc) | | 117,553 | 117,172 | 78,820 | 111,621 | 112,688 | 109,931 | 76,118 |
| Mz (conv.gpc) | | 270,191 | 277,755 | 186,520 | 258,547 | 254,301 | 197,425 | 155,254 |
| Mw/Mn (conv.gpc) | | 3.57 | 3.49 | 3.89 | 3.29 | 3.25 | 2.41 | 2.89 |

(continued)

| | Unit | Inv. First 1 | Inv. First 2 | Inv. First 3 | Comp. First A | Comp. First B | Comp. First C | Comp. First D |
|---|---|---|---|---|---|---|---|---|
| Mz/Mw (conv.gpc) | | 2.30 | 2.37 | 2.37 | 2.32 | 2.26 | 1.80 | 2.04 |
| Eta* (0.1 rad/s) | Pa•S | 9,496 | 11,231 | 1,997 | 10,342 | 11,929 | 6,975 | 2,057 |
| Eta* (1.0 rad/s) | Pa•S | 7,693 | 8,455 | 1,920 | 7,313 | 7,942 | 6,472 | 1,908 |
| Eta* (10 rad/s) | Pa•S | 4,706 | 4,977 | 1,527 | 4,337 | 4,586 | 5,071 | 1,473 |
| Eta* (100 rad/s) | Pa•S | 1,778 | 1,893 | 792 | 1,769 | 1,873 | 2,415 | 834 |
| Eta*0.1/ Eta*100 | | 5.34 | 5.93 | 2.52 | 5.85 | 6.37 | 2.89 | 2.47 |
| Eta zero | Pa•S | 11,210 | 13,947 | 2,142 | 12,994 | 15,661 | 7,748 | 2,176 |
| MWCDI | | 2.64 | 2.27 | 1.56 | 0.65 | 0.79 | -0.06 | -0.54 |
| Vinyls | Per 1000 total Carbons | 134 | 179 | 115 | 157 | 148 | 69 | 56 |
| ZSVR | | 1.53 | 1.92 | 1.25 | 2.13 | 2.49 | 1.35 | 1.45 |

EXAMPLE 2:

**Inventive Compositions 4 and 5**

Inventive compositions 4 and 5 each contain an ethylene-octene copolymer. Inventive compositions 4 and 5 were prepared in the same manner and using the same catalyst system as inventive compositions 1-3, with the exception of the polymerization conditions which are reported in Table 3.

Table 3: Polymerization Conditions (Rx1 = reactor 1; Rx2 = reactor 2)

| Sample # | Units | Inv. Comp. 4 | Inv. Comp. 5 |
|---|---|---|---|
| Reactor Configuration | | Dual Series | Dual Series |
| Comonomer | | 1 -octene | 1-octene |
| *REACTOR FEEDS* | | | |
| First Reactor Total Solvent Flow | lb/hr | 1323 | 957 |
| First Reactor Total Ethylene Flow | lb/hr | 228 | 200 |
| First Reactor Total Comonomer Flow | lb/hr | 86 | 71 |
| First Reactor Hydrogen Feed Flow | SCCM | 6379 | 4578 |
| Second Reactor Total Solvent Flow | lb/hr | 525 | 464 |
| Second Reactor Total Ethylene Flow | lb/hr | 201 | 211 |
| Second Reactor Total Comonomer Flow | lb/hr | 12 | 13 |
| Second Reactor Hydrogen Feed Flow | SCCM | 4392 | 2233 |
| *REACTION* | | | |
| First Reactor Control Temperature | °C | 165 | 165 |
| First Reactor Ethylene Conversion | % | 89.0 | 92.0 |

(continued)

| | | | |
|---|---|---|---|
| *REACTION* | | | |
| First Reactor Viscosity | cP | 402 | 1121 |
| Second Reactor Control | °C | 195 | 195 |
| Temperature | | | |
| Second Reactor Ethylene Conversion | % | 86.2 | 84.7 |
| Second Reactor Viscosity | cP | 219 | 524.7 |
| *CATALYST* | | | |
| First Reactor Catalyst | type | CAT 1 | CAT 1 |
| First Reactor Catalyst Efficiency | g polymer per g catalyst metal | 617060 | 1204000 |
| First Reactor Cocatalyst (Cocat. 2) to Catalyst Metal Molar Ratio | Ratio | 1.2 | 1.2 |
| First Reactor Cocatalyst (Cocat. 1) to Catalyst Metal Molar Ratio | Ratio | 18.0 | 50.0 |
| Second Reactor Catalyst Efficiency | g polymer per g catalyst metal | 354157 | 422627 |
| Second Reactor Al to Ti Molar Ratio | Ratio | 4.0 | 4.0 |
| * solvent = ISOPAR E | | | |

A breathable film was formed from Inventive Composition 4. Inventive Composition 4 was compounded using a Farrel continuous mixer with underwater pelletization. The amounts of the components that were compounded into pellets are shown in Table 4:

Table 4

| Component | Weight Percent |
|---|---|
| Inventive Composition 4 | 50% |
| Calcium Carbonate | 45% |
| LDPE | 5% |

The Calcium Carbonate was Imerys FilmLink® 500, which is commercially available from Imerys Carbonates. The LDPE was DOW™ LDPE 640I, which is commercially available from The Dow Chemical Company.

Films were fabricated on a 5 layer Egan Davis Standard coextrusion cast film line. The cast line consisted of three 2-1/2" and two 2" 30:1 LID Egan Davis Standard MAC extruders which are air cooled. All extruders had moderate work DSB (Davis Standard Barrier) type screws. Equipment specifications included a Cloeren 5 layer dual plane feed block and a Cloeren 36" Epich II autogage 5.1 die. The primary chill roll had a matte finish and was 40" outer diameter (O.D.) x 40" long with a 30-40 RMS surface finish for improved release characteristics. The secondary chill roll was 20" O.D. x 40" long with a 2-4 RMS surface for improved web tracking. Both the primary and secondary chill rolls had chilled water circulating through them to provide quenching. A Scantech X-ray gauge sensor for gauge thickness and automatic gauge control was available if needed. Films were fabricated at following conditions: Melt temperature = 400°F; Temperature profile (B1 300°F, B2 400°F, B3-5 400°F, Screen 400°F, Adaptor 400°F, Die all zones 400°F), Throughput rate = 200-270 lb/hr, Chill roll temperature = 70° F, Cast roll temperature = 70° F, Air knife = 7.4" $H_2O$, Vacuum box=OFF, Die gap = 20-25 mil, and Air gap = 4".

Each film was oriented using a Machine Direction Orientation (MDO) System, such as those available from Marshall and Williams Plastics and Parkinson Technologies, Inc. The film was oriented in the machine direction using a series of rollers at the same or different speeds to impart orientation. The film roll width on the feed roller was 24 inches with a thickness of 2.0 mil. The rollers were kept at the following temperatures: preheat roller 1 (165° F), preheat roller 2 (165° F), slow draw roller (185° F), fast draw roller (175° F), anneal roller (165° F) and cold roller (140° F). The speed of the slow draw roll was 5.1 feet per minute, and the speed of the fast draw roll was 19.1 feet per minute to provide a draw ratio of 3.75. The exit width of the film roll was 21.6 inches with a thickness of 0.7 - 0.8 mil. The temperature profile used in the MDO System is shown in Table 5:

Table 5

| Preheat 1 | Preheat 2 | Slow Draw | Fast Draw | Anneal | Cool |
|---|---|---|---|---|---|
| 165° F | 165° F | 185° F | 175° F | 165° F | 140° F |

Various film properties of the oriented film were measured. The number of samples, average values, and standard deviations are shown in Table 6:

Table 6

| Property | # of Samples | Average ± Std. Dev. |
|---|---|---|
| Elmendorf Tear, Machine Direction (gram force) | 15 | 3.6 ± 1.2 |
| Elmendor Tear, Cross Direction (gram force) | 15 | 525 ± 81 |
| Break Stress, Machine Direction (psi) | 5 | 4835 ± 1993 |
| Break Stress, Cross Direction (psi) | 5 | 954 ± 41 |
| Break Strain, Machine Direction (%) | 5 | 51 ±9 |
| Break Strain, Cross Direction (%) | 5 | 493 ± 25 |
| Puncture Strength (ft*lb/$in^3$) | 5 | 7.6 ± 1.9 |
| WVTR (g/$m^2$/day) | 2 | 2350 ± 44 |

EXAMPLE 3:

The stretch windows of Inventive Composition 4 and DOWLEX™ 2047G Polyethylene resin (commercially available from The Dow Chemical Company) were determined using MDO conditions as described above with modification of the roll temperatures as shown in Table 7:

Table 7

| Preheat 1 | Preheat 2 | Slow Draw | Fast Draw | Anneal | Cool |
|---|---|---|---|---|---|
| 165° F | 185° F | 205° F | 195° F | 165° F | 140° F |

The stretch windows are shown in Table 8:

Table 8

| Resin | Minimum Draw Ratio | Maximum Draw Ratio |
|---|---|---|
| Inventive Composition 4 | 3.25 | 5.75 |
| DOWLEX™ 2047G | 3.50 | 5.25 |

The minimum draw ratio was the draw ratio where haze variations (tiger striping) were visually absent from the film, and the maximum draw ratio was the draw ratio where pinholes or breaks in the film web were observed.

EXAMPLE 4:

**Inventive Compositions 6 and 7**

Inventive compositions 6 and 7 each contain an ethylene-octene copolymer. Inventive compositions 6 and 7 were prepared in the same manner and using the same catalyst system as inventive compositions 1-3, with the exception of the polymerization conditions which are reported in Table 9.

Table 9: Polymerization Conditions (Rx1 = reactor 1; Rx2 = reactor 2)

| Sample # | Units | Inv. Comp. 6 | Inv. Comp. 7 |
|---|---|---|---|
| Reactor Configuration | | Dual Series | Dual Series |
| Comonomer | | 1 -octene | 1-octene |
| *REACTOR FEEDS* | | | |
| First Reactor Total Solvent Flow | lb/hr | 965 | 724 |
| First Reactor Total Ethylene Flow | lb/hr | 201 | 151 |
| First Reactor Total Comonomer Flow | lb/hr | 49 | 23 |
| First Reactor Hydrogen Feed Flow | SCCM | 6092 | 4057 |
| Second Reactor Total Solvent Flow | lb/hr | 492 | 641 |
| Second Reactor Total Ethylene Flow | lb/hr | 223 | 291 |
| Second Reactor Total Comonomer Flow | lb/hr | 10 | 8 |
| Second Reactor Hydrogen Feed Flow | SCCM | 1786 | 4002 |
| *REACTION* | | | |
| First Reactor Control Temperature | °C | 160 | 160 |
| First Reactor Ethylene Conversion | % | 91.6 | 93.0 |
| First Reactor Viscosity | cP | 1620 | 2186 |
| Second Reactor Control Temperature | °C | 195 | 195 |
| Second Reactor Ethylene Conversion | % | 85.8 | 88.3 |
| Second Reactor Viscosity | cP | 579.2 | 775.7 |
| *CATALYST* | | | |
| First Reactor Catalyst | type | CAT 1 | CAT 1 |
| First Reactor Catalyst Efficiency | g polymer per g catalyst metal | 2610844 | 2461080 |
| First Reactor Cocatalyst (Cocat. 2) to Catalyst Metal Molar Ratio | Ratio | 1.3 | 1.2 |
| First Reactor Cocatalyst (Cocat. 1) to Catalyst Metal Molar Ratio | Ratio | 50.0 | 50.0 |
| Second Reactor Catalyst Efficiency | g polymer per g catalyst metal | 622016 | 741104 |
| Second Reactor Al to Ti Molar Ratio | Ratio | 4.0 | 4.0 |

Properties of the Inventive Compositions were measured, and are reported in Table 10.

Table 10: Properties of Inventive Compositions

| | Unit | Inv. Comp. 6 | Inv. Comp. 7 | |
|---|---|---|---|---|
| Density | g/cc | 0.9244 | 0.9351 | |
| $I_2$ | g/10 min | 2.31 | 2.61 | |
| $I_{10}/I_2$ | | 7.5 | 7.5 | |
| 7.0-1.2xlog(I2) | | 6.6 | 6.5 | |
| Mn (conv.gpc) | g/mol | 30,905 | 29,269 | |
| Mw (conv.gpc) | g/mol | 93,984 | 88,988 | |
| Mz (conv.gpc) | g/mol | 223,635 | 204,901 | |
| Mw/Mn (conv.gpc) | | 3.04 | 3.04 | |

(continued)

| | Unit | Inv. Comp. 6 | Inv. Comp. 7 | |
|---|---|---|---|---|
| Mz/Mw (conv.gpc) | | 2.38 | 2.30 | |
| Eta* (0.1 rad/s) | Pa•S | 3,876 | 3,308 | |
| Eta* (1.0 rad/s) | Pa•S | 3,356 | 2,869 | |
| Eta* (10 rad/s) | Pa•S | 2,337 | 2,028 | |
| Eta* (100 rad/s) | Pa•S | 1,107 | 1,000 | |
| Eta*0.1/ Eta*100 | | 3.50 | 3.31 | |
| MWCDI | | 1.80 | 1.23 | |

Breathable films can be formed from Inventive Compositions 6 and 7. That which is claimed:

## Claims

1. A breathable film comprising a first composition, wherein the first composition comprises at least one ethylene-based polymer and wherein the first composition comprises a MWCDI value greater than 0.9, and a melt index ratio (I10/I2) that meets the following equation: $I10/I2 \geq 7.0 - 1.2 \times \log (I2)$; wherein the first composition has a melt index ratio I10/I2 less than, or equal to, 9.2; wherein the breathable film further comprises 40 to 65 weight percent of a mineral filler; wherein MWCDI is Molecular Weighted Comonomer Distribution Index and is measured as described in the description; and wherein melt indices I2 and I10 are measured in accordance to ASTM D-1238 (method B) at 190°C and at 2.16 kg and 10 kg load, respectively.

2. The breathable film of claim 1, wherein the first composition has a MWCDI value less than, or equal to, 10.0.

3. The breathable film of any of the preceding claims, wherein the first composition has a density of 0.910 to 0.940 g/$cm^3$ and a melt index (I2) of 0.5 to 30 g/10 minutes.

4. The breathable film of any of the preceding claims, wherein the film has a basis weight of 10 to 20 g/$m^2$.

5. The breathable film of any of the preceding claims, further comprising a second polymer, and wherein the second polymer is selected from the following: a LLDPE, a VLDPE, a LDPE, a MDPE, a HDPE, a HMWHDPE, a propylene-based polymer, a polyolefin plastomer, a polyolefin elastomer, an olefin block copolymer, an ethylene vinyl acetate, an ethylene acrylic acid, an ethylene methacrylic acid, an ethylene methyl acrylate, an ethylene ethyl acrylate, an ethylene butyl acrylate, a polyisobutylene, a maleic anhydride-grafted polyolefin, an ionomer of any of the foregoing, or a combination thereof.

6. The breathable film of any of the preceding claims, wherein the breathable film comprises a first layer comprising the first composition, and a second layer, wherein the second layer comprises a polymer selected from the following: the first composition, a LLDPE, a VLDPE, a LDPE, a MDPE, a HDPE, a HMWHDPE, a propylene-based polymer, a polyolefin plastomer, a polyolefin elastomer, an olefin block copolymer, an ethylene vinyl acetate, an ethylene acrylic acid, an ethylene methacrylic acid, an ethylene methyl acrylate, an ethylene ethyl acrylate, an ethylene butyl acrylate, a polyisobutylene, a maleic anhydride-grafted polyolefin, an ionomer of any of the foregoing, or a combination thereof.

7. The breathable film of any of the preceding claims, wherein the breathable film comprises at least 30 weight percent of the first composition.

8. The breathable film of any of the preceding claims, wherein the breathable film exhibits a water vapor transmission rate of at least 500 g/$m^2$-day-atm and up to 10,000 g/$m^2$-day-atm.

9. The breathable film of any of the preceding claims, wherein the breathable film is oriented in at least one of the machine direction and the cross direction.

10. The breathable film of any of the preceding claims, wherein the breathable film is oriented in the machine direction and

the cross direction.

11. The breathable film of any of the preceding claims, wherein the first composition has a MWCDI value greater than 1.2.

12. A laminate comprising the breathable film of any of the preceding claims.

13. The laminate of claim 12, further comprising a non-woven material.

14. An article comprising the laminate of claim 12 or claim 13.

15. An article comprising the breathable film of any of claims 1-11.

## Patentansprüche

1. Atmungsaktiver Film, umfassend eine erste Zusammensetzung, wobei die erste Zusammensetzung mindestens ein Polymer auf Ethylenbasis umfasst und wobei die erste Zusammensetzung einen MWCDI-Wert größer als 0,9 und ein Schmelzindexverhältnis (110/12) umfasst, das die folgende Gleichung erfüllt: $110/12 \geq 7{,}0 - 1{,}2 \times \log(I2)$; wobei die erste Zusammensetzung ein Schmelzindexverhältnis 110/12 kleiner als oder gleich 9,2 aufweist; wobei der atmungsaktive Film ferner zu 40 bis 65 Gewichtsprozent einen mineralischen Füllstoff umfasst; wobei MWCDI ein molekulargewichteter Comonomer-Verteilungsindex ist und wie in der Beschreibung beschrieben gemessen wird; und wobei die Schmelzindizes I2 und 110 gemäß ASTM D-1238 (Verfahren B) bei 190 °C und einer Belastung von 2,16 kg bzw. 10 kg gemessen werden.

2. Atmungsaktiver Film nach Anspruch 1, wobei die erste Zusammensetzung einen MWCDI-Wert kleiner als oder gleich 10,0 aufweist.

3. Atmungsaktiver Film nach einem der vorstehenden Ansprüche, wobei die erste Zusammensetzung eine Dichte von 0.910 bis 0.940 $g/cm^3$ und einen Schmelzindex (I2) von 0,5 bis 30 g/10 Minuten aufweist.

4. Atmungsaktiver Film nach einem der vorstehenden Ansprüche, wobei der Film ein Basisgewicht von 10 bis 20 $g/m^2$ aufweist.

5. Atmungsaktiver Film nach einem der vorstehenden Ansprüche, ferner umfassend ein zweites Polymer und wobei das zweite Polymer aus den Folgenden ausgewählt ist: einem LLDPE, einem VLDPE, einem LDPE, einem MDPE, einem HDPE, einem HMWHDPE, einem Polymer auf Propylenbasis, einem Polyolefinplastomer, einem Polyolefinelastomer, einem Olefin-Blockcopolymer, einem Ethylenvinylacetat, einer Ethylenacrylsäure, einer Ethylenmethacrylsäure, einem Ethylenmethylacrylat, einem Ethylenethylacrylat, einem Ethylenbutylacrylat, einem Polyisobutylen, einem Maleinsäureanhydrid-gepfropften Polyolefin, einem Ionomer eines beliebigen der Vorstehenden oder einer Kombination davon.

6. Atmungsaktiver Film nach einem der vorstehenden Ansprüche, wobei der atmungsaktive Film eine erste Schicht, umfassend die erste Zusammensetzung, und eine zweite Schicht umfasst, wobei die zweite Schicht ein Polymer umfasst, das aus den Folgenden ausgewählt ist: der ersten Zusammensetzung, einem LLDPE, einem VLDPE, einem LDPE, einem MDPE, einem HDPE, einem HMWHDPE, einem Polymer auf Propylenbasis, einem Polyolefinplastomer, einem Polyolefinelastomer, einem Olefin-Blockcopolymer, einem Ethylenvinylacetat, einer Ethylenacrylsäure, einer Ethylenmethacrylsäure, einem Ethylenmethylacrylat, einem Ethylenethylacrylat, einem Ethylenbutylacrylat, einem Polyisobutylen, einem Maleinsäureanhydrid-gepfropften Polyolefin, einem Ionomer eines beliebigen der Vorstehenden oder einer Kombination davon.

7. Atmungsaktiver Film nach einem der vorstehenden Ansprüche, wobei der atmungsaktive Film mindestens zu 30 Gewichtsprozent die erste Zusammensetzung umfasst.

8. Atmungsaktiver Film nach einem der vorstehenden Ansprüche, wobei der atmungsaktive Film eine Wasserdampfdurchlässigkeitsrate von mindestens 500 $g/m^2$-Tag-atm und bis zu 10.000 $g/m^2$-Tag-atm aufweist.

9. Atmungsaktiver Film nach einem der vorstehenden Ansprüche, wobei der atmungsaktive Film in mindestens einer der Maschinenrichtung und der Querrichtung orientiert ist.

**10.** Atmungsaktiver Film nach einem der vorstehenden Ansprüche, wobei der atmungsaktive Film in der Maschinenrichtung und der Querrichtung orientiert ist.

**11.** Atmungsaktiver Film nach einem der vorstehenden Ansprüche, wobei die erste Zusammensetzung einen MWCDI-Wert größer als 1,2 aufweist.

**12.** Laminat, umfassend den atmungsaktiven Film nach einem der vorstehenden Ansprüche.

**13.** Laminat gemäß Anspruch 12, ferner umfassend ein Vliesmaterial.

**14.** Erzeugnis, umfassend das Laminat nach Anspruch 12 oder 13.

**15.** Erzeugnis, umfassend den atmungsaktiven Film nach einem der Ansprüche 1 bis 11.

## Revendications

**1.** Film respirant comprenant une première composition, dans lequel la première composition comprend au moins un polymère à base d'éthylène et dans lequel la première composition comprend une valeur MWCDI supérieure à 0,9 et un rapport d'indice de fusion (I10/I2) qui satisfait à l'équation suivante : $110/12 \geq 7{,}0 - 1{,}2 \times \log (I2)$ ; dans lequel la première composition a un rapport d'indice de fusion 110/12 inférieur ou égal à 9,2 ; dans lequel le film respirant comprend en outre 40 à 65 pour cent en poids d'une charge minérale ; dans lequel MWCDI est un indice de distribution des monomères pondéré par poids moléculaire et est mesuré comme décrit dans la description ; et dans lequel les indices de fusion I2 et I10 sont mesurés conformément à la norme ASTM D-1238 (méthode B) à 190 °C et à une charge de 2,16 kg et 10 kg, respectivement.

**2.** Film respirant selon la revendication 1, dans lequel la première composition a une valeur MWCDI inférieure ou égale à 10,0.

**3.** Film respirant selon l'une quelconque des revendications précédentes, dans lequel la première composition a une masse volumique de 0,910 à 0,940 g/cm$^3$ et un indice de fusion (I2) de 0,5 à 30 g/10 minutes.

**4.** Film respirant selon l'une quelconque des revendications précédentes, dans lequel le film a une masse surfacique de 10 à 20 g/cm$^2$.

**5.** Film respirant selon l'une quelconque des revendications précédentes, comprenant en outre un second polymère, et dans lequel le second polymère est choisi parmi ce qui suit : un LLDPE, un VLDPE, un LDPE, un MDPE, un HDPE, un HMWHDPE, un polymère à base de propylène, un plastomère polyoléfinique, un élastomère polyoléfinique, un copolymère séquencé oléfinique, un éthylène-acétate de vinyle, un éthylène-acide acrylique, un éthylène-acide méthacrylique, un éthylène-acrylate de méthyle, un éthylène-acrylate d'éthyle, un éthylène-acrylate de butyle, un polyisobutylène, une polyoléfine greffée par anhydride maléique, un ionomère de l'un quelconque de ce qui précède, ou une combinaison de ceux-ci.

**6.** Film respirant selon l'une quelconque des revendications précédentes, dans lequel le film respirant comprend une première couche comprenant la première composition, et une seconde couche, dans lequel la seconde couche comprend un polymère choisi parmi ce qui suit : la première composition, un LLDPE, un VLDPE, un LDPE, un MDPE, un HDPE, un HMWHDPE, un polymère à base de propylène, un plastomère polyoléfinique, un élastomère polyoléfinique, un copolymère séquencé oléfinique, un éthylène-acétate de vinyle, un éthylène-acide acrylique, un éthylène-acide méthacrylique, un éthylène-acrylate de méthyle, un éthylène-acrylate d'éthyle, un éthylène-acrylate de butyle, un polyisobutylène, une polyoléfine greffée par anhydride maléique, un ionomère de l'un quelconque de ce qui précède, ou une combinaison de ceux-ci.

**7.** Film respirant selon l'une quelconque des revendications précédentes, dans lequel le film respirant comprend au moins 30 pour cent en poids de la première composition.

**8.** Film respirant selon l'une quelconque des revendications précédentes, dans lequel le film respirant présente un taux de transmission de vapeur d'eau d'au moins 500 g/m$^2$-jour-atm et jusqu'à 10 000 g/m$^2$-jour-atm.

**9.** Film respirant selon l'une quelconque des revendications précédentes, dans lequel le film respirant est orienté dans au moins l'un parmi le sens machine et le sens travers.

**10.** Film respirant selon l'une quelconque des revendications précédentes, dans lequel le film respirant est orienté dans le sens machine et le sens travers.

**11.** Film respirant selon l'une quelconque des revendications précédentes, dans lequel la première composition a une valeur MWCDI supérieure à 1,2.

**12.** Stratifié comprenant le film respirant selon l'une quelconque des revendications précédentes.

**13.** Stratifié selon la revendication 12, comprenant en outre un matériau non tissé.

**14.** Article comprenant le stratifié selon la revendication 12 ou la revendication 13.

**15.** Article comprenant le film respirant selon l'une quelconque des revendications 1 à 11.

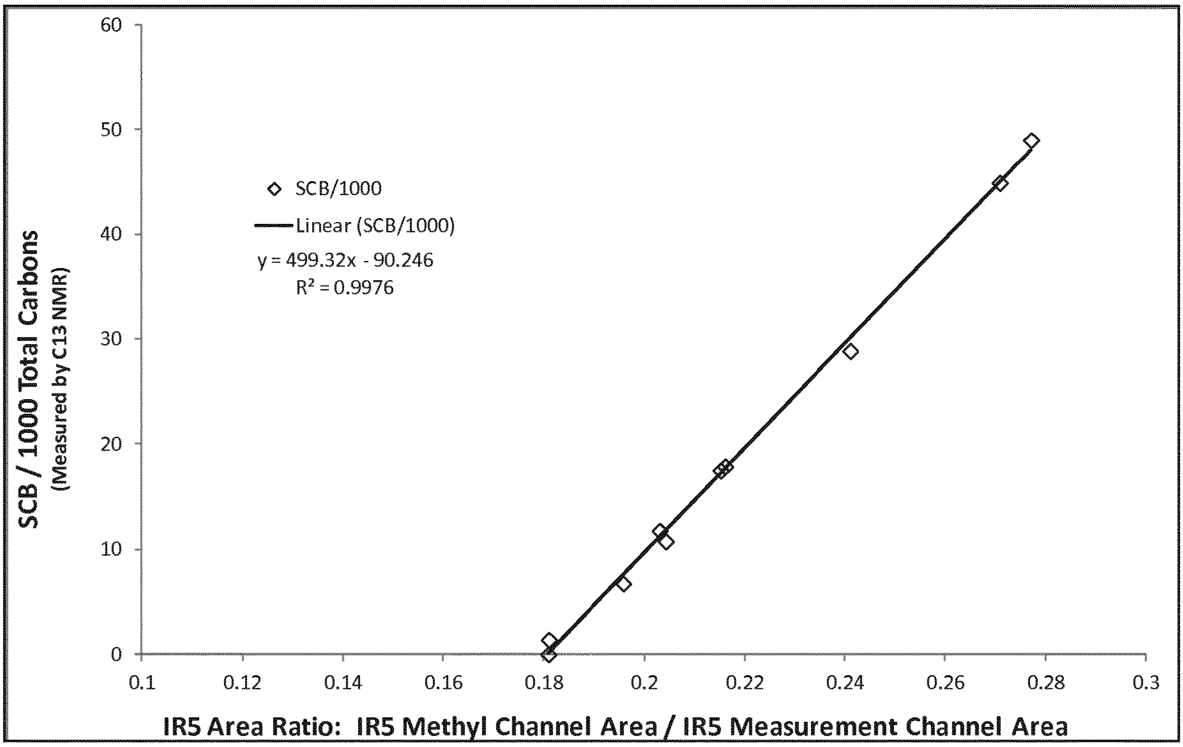


FIGURE 1

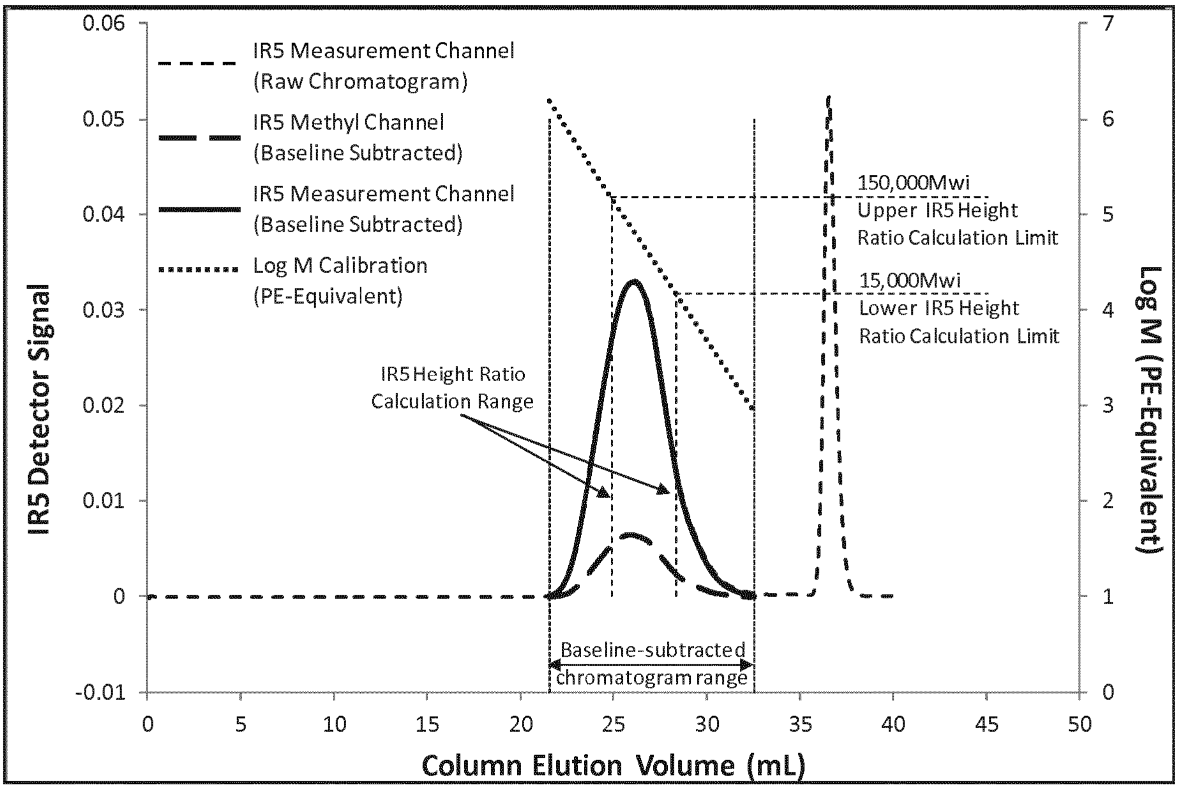


FIGURE 2

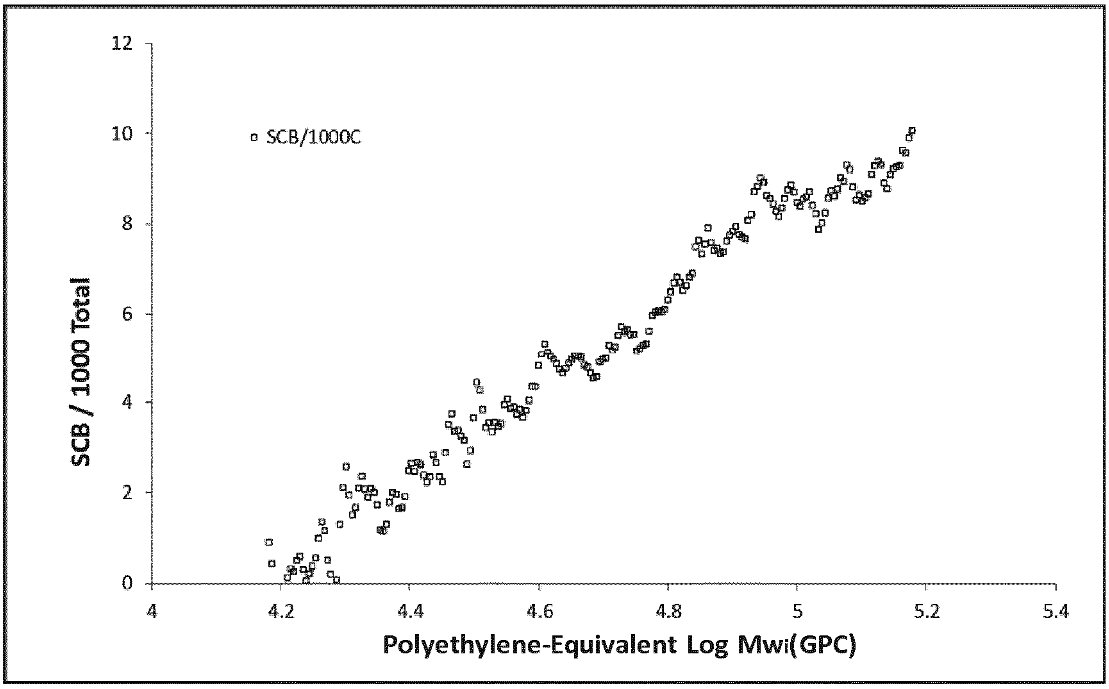


FIGURE 3

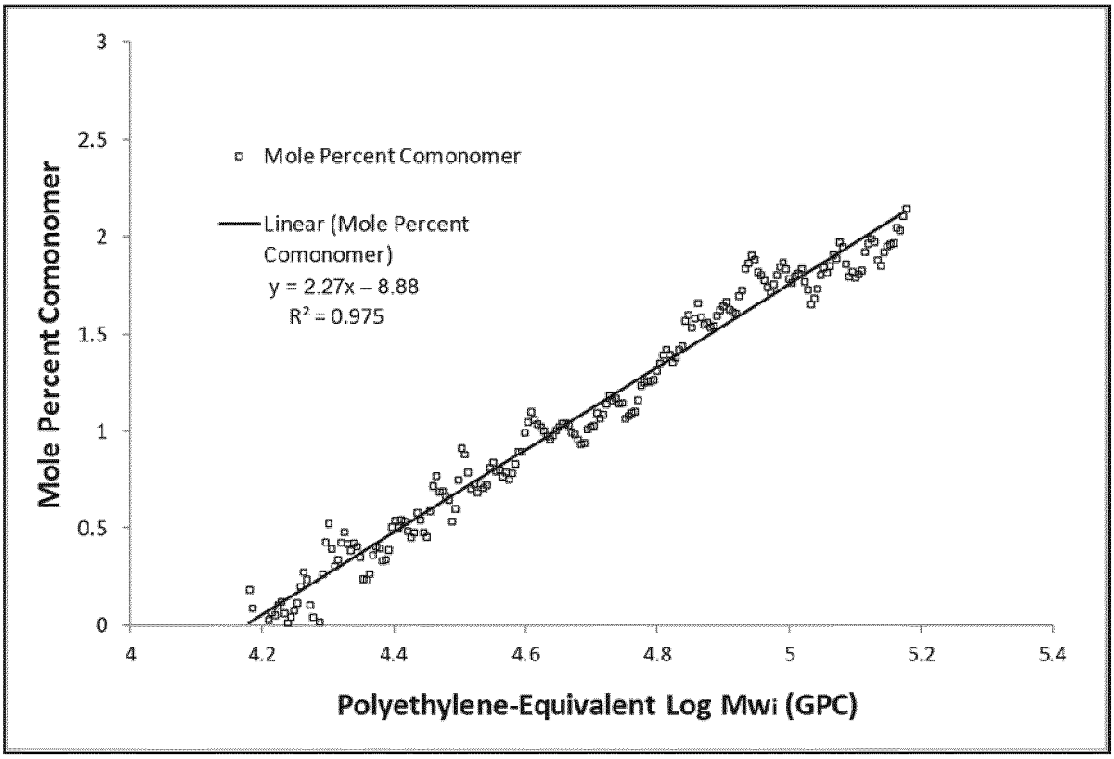


FIGURE 4

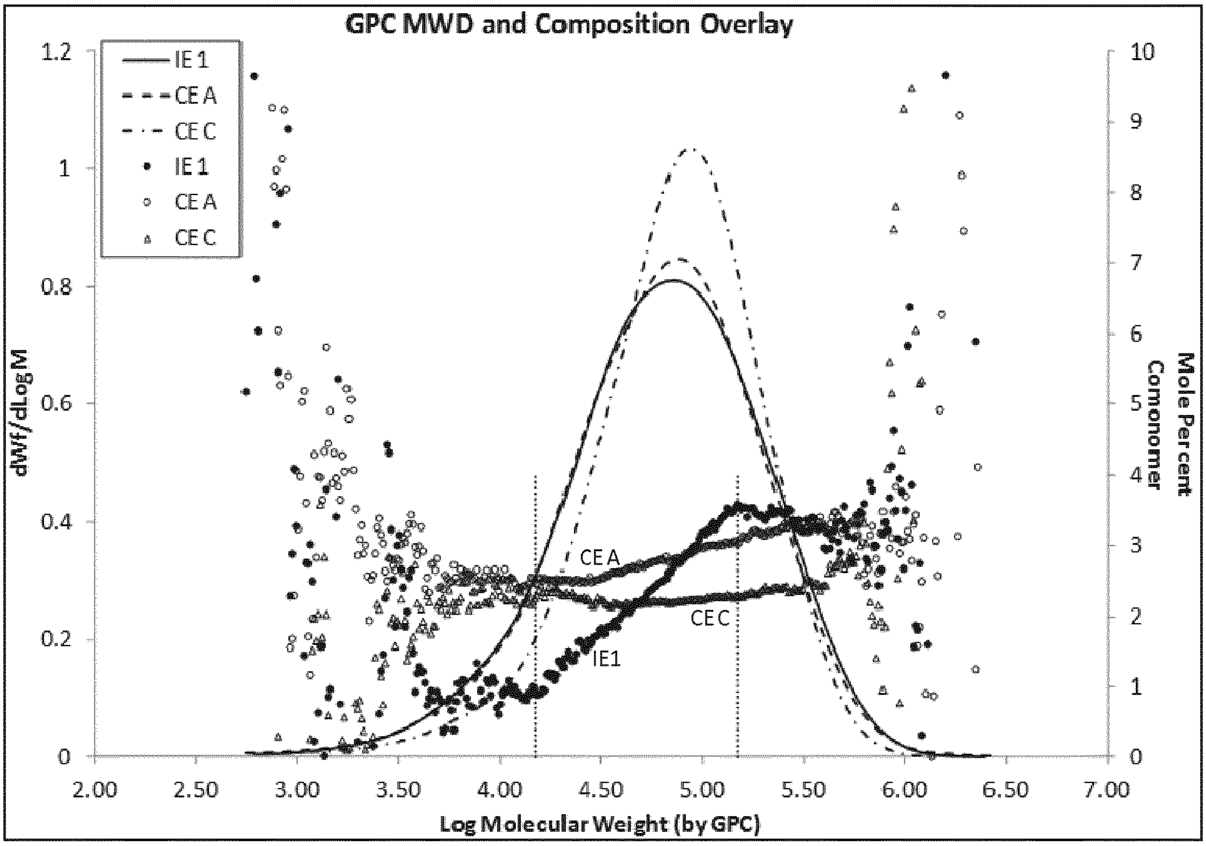


FIGURE 5

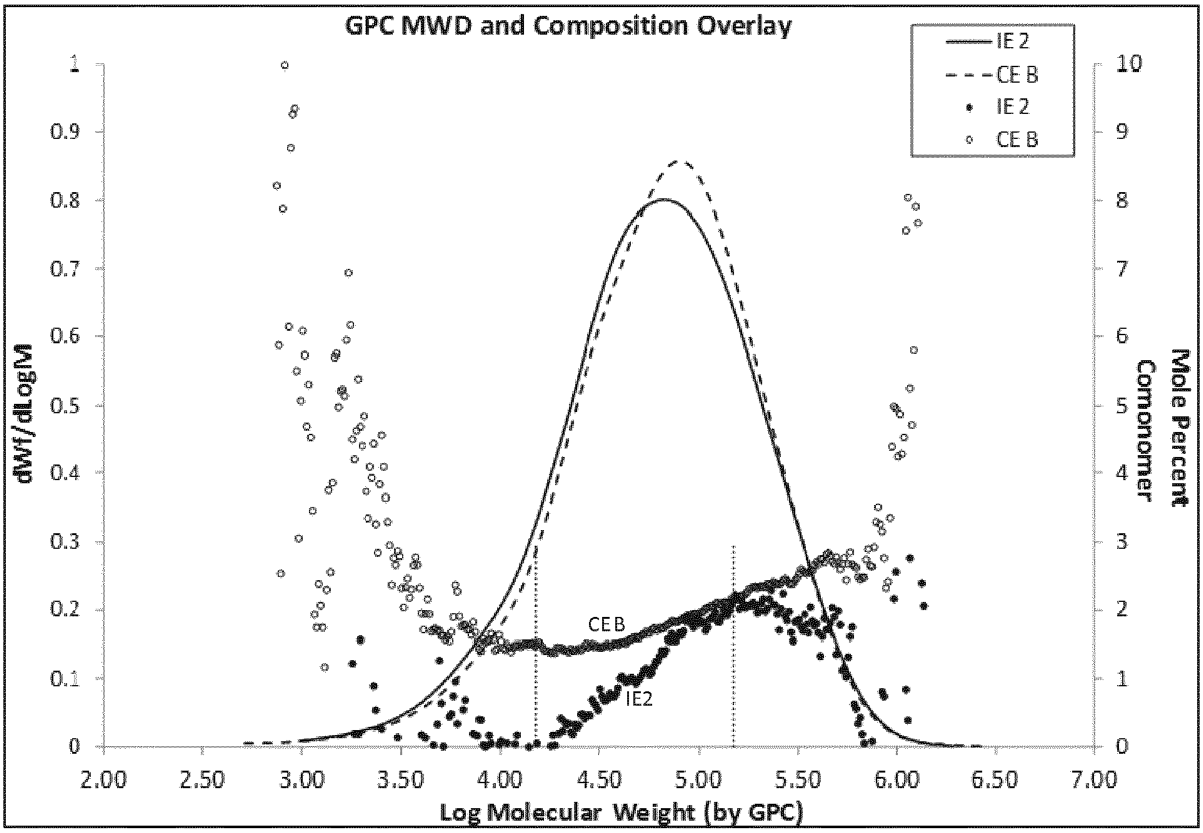


FIGURE 6

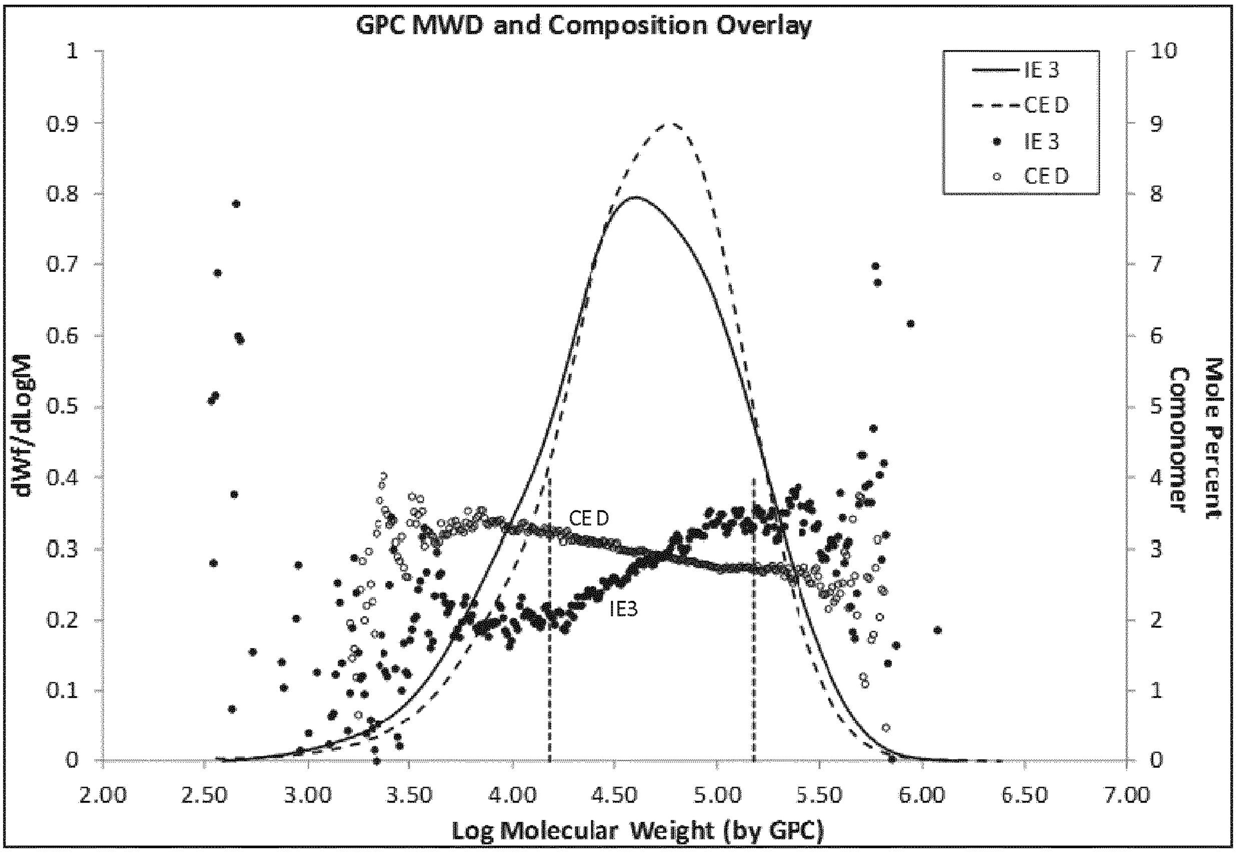
GPC MWD and Composition Overlay
dWf/dLogM
Mole Percent Comonomer
Log Molecular Weight (by GPC)
IE 3
CE D
IE 3
CE D
CE D
IE3
1
0.9
0.8
0.7
0.6
0.5
0.4
0.3
0.2
0.1
0
10
9
8
7
6
5
4
3
2
1
0
2.00
2.50
3.00
3.50
4.00
4.50
5.00
5.50
6.00
6.50
7.00

FIGURE 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4612300 A
- US 4314912 A
- US 4547475 A
- US 6103657 A
- US 5064802 A
- US 5153157 A
- US 5296433 A
- US 5321106 A
- US 5350723 A
- US 5425872 A
- US 5625087 A
- US 5721185 A
- US 5783512 A
- US 5883204 A
- US 5919983 A
- US 6696379 B
- US 7163907 B
- US 6515155 B1
- US 4116892 A
- US 4153751 A
- US 5977251 A


### Non-patent literature cited in the description


- **WU et al.** Novel Microporous Films and Their Composites. *Journal of Engineered Fibers and Fabrics*, 2007, vol. 2 (1), 49-59
- **P.C. WU et al.** Novel Microporous Films and Their Composites. *Journal of Engineered Fibers and Fabrics*, 2007, vol. 2 (1), 49-59
- **WILLIAMS** ; **WARD**. *J. Polym. Sci., Polym. Letters*, 1968, vol. 6, 621
- **KARJALA et al.** Detection of Low Levels of Long-chain Branching in Polyolefins. *Annual Technical Conference - Society of Plastics Engineers*, 2008, 887-891
- **BUSICO, V. et al.** *Macromolecules*, 2005, vol. 38, 6988
- NMR and Macromolecules. **J. C. RANDALL et al.** ACS Symposium. Am. Chem. Soc., 1984
- **J. C. RANDALL**. Polymer Sequence Determination. Academic Press, 1977
- **WILLIAMS** ; **WARD**. *J. Polym. Sci., Polym. Let.*, 1968, vol. 6, 621